# EUROPÄISCHE PATENTSCHRIFT

(11) **EP 2 139 835 B1**
(45) Veröffentlichungstag und Bekanntmachung des Hinweises auf die Patenterteilung: **10.05.2017**
(21) Anmeldenummer: 08736312.3
(22) Anmeldetag: 17.04.2008
(51) Int. Cl.: C07C 45/62, C07C 47/21, C07C 29/56, C07C 29/17, C07C 35/12

(54) **VERFAHREN ZUR HERSTELLUNG OPTISCH AKTIVER CARBONYLVERBINDUNGEN**
METHOD FOR SYNTHESIZING OPTICALLY ACTIVE CARBONYL COMPOUNDS
PROCÉDÉ POUR PRODUIRE DES COMPOSÉS CARBONYLE OPTIQUEMENT ACTIFS

(30) Priorität: 25.04.2007 EP 07106922
(43) Veröffentlichungstag der Anmeldung: 06.01.2010
(73) Patentinhaber: BASF SE, 67056 Ludwigshafen am Rhein (DE)
(72) Erfinder: SCHMIDT-LEITHOFF, Joachim, 79098 Freiburg (DE); JÄKEL, Christoph, 67117 Limburgerhof (DE); PACIELLO, Rocco, 67098 Bad Dürkheim (DE)
(74) Vertreter: BASF IP Association
(86) Internationale Anmeldenummer: PCT/EP2008/054644
(87) Internationale Veröffentlichungsnummer: WO 2008/132057

(56) Entgegenhaltungen:
- WO-A-2006/040096

## Beschreibung

Die vorliegende Erfindung betrifft ein kontinuierliches Verfahren zur Herstellung optisch aktiver Carbonylverbindungen durch asymmetrische Hydrierung a,β-ungesättigter Carbonylverbindungen in Gegenwart von im Reaktionsgemisch löslichen, optisch aktiven Übergangsmetall-Katalysatoren, die mindestens einen Kohlenmonoxid-Liganden aufweisen. Speziell betrifft die vorliegende Erfindung ein Verfahren zur Herstellung optisch aktiver Aldehyde oder Ketone, insbesondere Citronellal durch asymmetrische Hydrierung der entsprechenden optisch aktiven α,β-ungesättigten Aldehyde oder Ketone in Gegenwart von Kohlenmonoxid.

Viele optisch aktive Aldehyde und Ketone stellen wertvolle Zwischenstoffe zur Synthese höher veredelter chiraler Wert- und Wirkstoffe dar und sind ihrerseits oft begehrte Riech- und Aromastoffe.

Die EP-A 0 000 315 betrifft ein Verfahren zur Herstellung von optisch aktivem Citronellal durch Hydrierung von Geranial oder Neral in Gegenwart eines im Reaktionssystem gelösten Katalysatorkomplexes aus Rhodium und einem chiralen Phosphin.

T.-P. Dang et al. beschreiben in J. Mol. Cat. 1982, 16, 51 - 59 die homogenkatalytische Hydrierung a,β-ungesättigter Aldhyde sowie die Anwendung des Verfahrens zur Herstellung von optisch aktivem Citronellal. Als Katalysatoren dienten dabei Komplexverbindungen aus einem Rhodiumcarbonyl und einem chiralen Diphosphin.

Auch Chapuis et al. erwähnen in Helv. Chim. Acta 2001, 84, 230 -242, Fußnote 4, die asymmetrische Hydrierung von Geranial bzw. Neral zu optisch aktivem Citronellal in Gegenwart eines Katalysators aus Rh₄(CO)₁₂ und (R,R)-Chiraphos ((2R, 3R)-2,3-bis(diphenylphosphino)butan).

Ein Problem bei der Durchführung mittels löslicher Katalysatoren katalysierter (homogenkatalytischer) Reaktionen besteht in der oft unzureichenden Stabilität der eingesetzten Katalysatorkomplexe bzw. der sich daraus bildenden katalytisch aktiven Metall- bzw. Übergangsmetall-Komplexverbindung. Vor dem Hintergrund des oft hohen Preises derartiger Katalysatoren bzw. Katalysatorvorstufen sind homogenkatalytische Reaktionen mit komplexen Übergangsmetallkatalysatoren nur in speziellen Fällen in wirtschaftlicher Weise im technischen Maßstab anwendbar.

Die JP-A 52078812 beschreibt ein Verfahren zur Hydrierung a,β-ungesättigter Aldehyde wie Crotonaldehyd, Zimtaldehyd oder α-Methylzimtaldehyd an homogenen Rh-Katalysatoren unter Hydroformylierungsbedingungen in Gegenwart eines Triarylphosphins, eines tertiären Amins in stöchiometrischer Menge und Kohlenmonoxid.

Die WO 2006/040096 offenbart ein Verfahren zur Herstellung optisch aktiver Carbonylverbindungen durch asymmetrische Hydrierung a,β-ungesättigter Carbonylverbindungen in Gegenwart von im Reaktionsgemisch löslichen, optisch aktiven Übergangsmetall-Katalysatoren, die mindestens einen Kohlenmonoxid-Liganden aufweisen, das dadurch gekennzeichnet ist, dass man den Katalysator mit einem Kohlenmonoxid und Wasserstoff enthaltenden Gasgemisch vorbehandelt und/oder die asymmetrische Hydrierung in Gegenwart von dem Reaktionsgemisch zusätzlich zugeführtem Kohlenmonoxid durchführt.

Nimmt man dabei die Reaktion so vor, dass man den Katalysator mit einem Kohlenmonoxid und Wasserstoff enthaltenden Gasgemisch vorbehandelt und die anschlie-ßende Hydrierung in Gegenwart von dem Reaktionsgemisch zusätzlich zugeführtem Kohlenmonoxid durchführt, ist die Kohlenmonoxidkonzentration des Reaktionsgemischs während der Hydrierung oft schwer zu kontrollieren. Zudem wird die Vorbehandlung in der Regel unter Einsatz deutlich höherer Kohlenmonoxid-Konzentrationen durchgeführt als die asymmetrische Hydrierung, so dass sich große Mengen Kohlenmonoxid, die aus der Vorbehandlung des Katalysators stammen, in die Hydrierung eingeschleppt können und sich dort unvorteilhaft auswirken.

Aufgabe der vorliegenden Erfindung war es demnach, das in der vorstehend genannten WO 2006/040096 offenbarte Verfahren dahingehend zu verbessern, dass die Vorbehandlung des Katalysators und die Durchführung der asymmetrischen Hydrierung auf verfahrenstechnisch einfache Weise unter den für die einzelnen Schritte jeweils optimalen Kohlenmonoxid-Konzentrationen durchgeführt werden können. Überdies soll das verbesserte Verfahren ermöglichen, die asymmetrische Hydrierung unter möglichst konstanter Kohlenmonoxid-Konzentration durchführen zu können.

Die Aufgabe wurde gelöst durch ein kontinuierliches Verfahren zur Herstellung optisch aktiver Carbonylverbindungen der Formel (I) 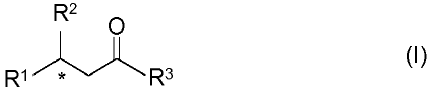 wobei die Reste
- R¹, R²: jeweils für einen unverzweigten, verzweigten oder cyclischen Alkylrest mit 1 bis 25 Kohlenstoffatomen, der gegebenenfalls eine oder mehrere ethylenische Doppelbindungen und/oder einen oder mehrere gleiche oder verschiedene Substituenten ausgewählt aus der Gruppe der Substituenten OR⁴, NR⁵R⁶, Halogen, C₆-C₁₀-Aryl und C₃-C₉-Hetaryl tragen kann und der gemeinsam mit R³ einen 5 bis 25-gliedrigen Ring bilden kann, steht, mit der Maßgabe, dass R¹ und R² verschieden sind
- R³: für Wasserstoff oder einen unverzweigten, verzweigten oder cyclischen Alkylrest mit 1 bis 25 Kohlenstoffatomen, der gegebenenfalls eine oder mehrere ethylenische Doppelbindungen und/oder eine oder mehrere gleiche oder verschiedene Substituenten ausgewählt aus der Gruppe der Substituenten OR⁴, NR⁵R⁶, Halogen, C₆-C₁₀- Aryl und C₃-C₉-Hetaryl tragen kann, oder für OR⁷ oder NR⁸R⁹ steht
wobei
- R⁴, R⁵, R⁶: jeweils unabhängig voneinander Wasserstoff, C₁-C₆-Alkyl, C₆-C₁₀- Aryl, C₇-C₁₂-Aralkyl oder C₇-C₁₂-Alkylaryl bedeuten und
- R⁵ und R⁶: gemeinsam auch eine Alkylenkette mit 2 bis 5 Kohlenstoffatomen, die durch N oder O unterbrochen sein kann, bedeuten können und
- R⁷: für einen unverzweigten, verzweigten oder cyclischen Alkylrest mit 1 bis 25 Kohlenstoffatomen, der gegebenenfalls eine oder mehrere ethylenische Doppelbindungen und eine oder mehrere gleiche oder verschiedene Substituenten ausgewählt aus der Gruppe der Substituenten OR⁴, NR⁵R⁶, Halogen, C₆-C₁₀-Aryl und C₃-C₉-Hetaryl tragen kann, steht und gemeinsam mit R¹ oder R² einen 5 bis 25-gliedrigen Ring bilden kann und
- R⁸: für einen unverzweigten, verzweigten oder cyclischen Alkylrest mit 1 bis 25 Kohlenstoffatomen, der gegebenenfalls eine oder mehrere ethylenische Doppelbindungen und eine oder mehrere gleiche oder verschiedene Substituenten ausgewählt aus der Gruppe der Substituenten OR⁴, NR⁵R⁶, Halogen, C₆-C₁₀-Aryl und C₃-C₉-Hetaryl tragen kann, stehen und gemeinsam mit R¹, R² oder R⁹ einen 5 bis 25-gliedrigen Ring bilden kann und
- R⁹: für Wasserstoff, C₁-C₆-Alkyl, C₆-C₁₀-Aryl, C₇-C₁₂-Aralkyl oder C₇-C₁₂-Alkylaryl steht oder zusammen mit R⁸ einen 5 bis 25-gliedrigen Ring bilden kann und
- *: ein asymmetrisches Kohlenstoffatom bezeichnet,
durch asymmetrische Hydrierung a,β-ungesättigter Aldehyde oder Ketone der Formel (II) 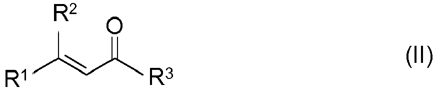 wobei die Reste R¹ bis R³ die oben angegebene Bedeutung besitzen,
in Gegenwart von im Reaktionsgemisch löslichen, optisch aktiven Übergangsmetall-Katalysatoren, die mindestens einen Kohlenmonoxid-Liganden aufweisen, wobei man zur Herstellung des jeweils einzusetzenden optisch aktiven, mindestens einen Kohlenmonoxid-Liganden aufweisenden Katalysators eine Katalysator-Vorstufe mit einem Kohlenmonoxid und Wasserstoff enthaltenden Gasgemisch vorbehandelt und die asymmetrische Hydrierung in Gegenwart von dem Reaktionsgemisch zusätzlich zugeführtem Kohlenmonoxid durchführt, indem man gemäß der aufeinanderfolgenden Schritte
a) die Vorbehandlung der Katalysator-Vorstufe mit einem Gasgemisch umfassend 30 bis 70 Vol.-% Kohlenmonoxid, 30 bis 70 Vol-% Wasserstoff und 0 bis 5 Vol.-% weiterer Gase, wobei sich die genannten Volumenanteile zu 100 Vol.-% ergänzen, bei einem Druck von 50 bis 90 bar durchführt,
b) von dem so erhaltenen Katalysator vor Einsatz in der asymmetrischen Hydrierung überschüssiges Kohlenmonoxid abtrennt, indem man den gemäß Schritt a) vorbehandelten Katalysator auf einen Druck im Bereich von 1 bis 3 bar entspannt und
c) die asymmetrische Hydrierung in Gegenwart von Wasserstoff mit einem Kohlenmonoxidgehalt von 100 bis 1200 ppm durchführt,
und das erhaltene Reaktionsprodukt vom Reaktionsgemisch entfernt wird und der zurückbleibende Katalysator nach der Vorbehandlung gemäß Schritt a) zu weiteren Umsetzungen genutzt wird.

Erfindungsgemäß hydriert man die zur Carbonylgruppe α,β-ständige ethylenische Doppelbindung in Gegenwart eines im Reaktionsgemisch löslichen, optisch aktiven Übergangsmetallkatalysators, der mindestens einen Kohlenmonoxid- bzw. CO-Liganden aufweist, zu einer Kohlenstoff-Kohlenstoff-Einfachbindung, wobei das dadurch in β-Position neu geschaffene tetraedrische Kohlenstoffatom asymmetrisch substituiert ist und in nicht-racemischer Form erhalten wird. Unter dem Begriff asymmetrische Hydrierung ist demgemäss im Rahmen der vorliegenden Erfindung eine Hydrierung zu verstehen, bei der die beiden enantiomeren Formen des Hydrierproduktes nicht zu gleichen Teilen erhalten werden.

Zur Herstellung des jeweils einzusetzenden optisch aktiven, mindestens einen Kohlenmonoxid-Liganden aufweisenden Katalysators führt man eine Vorbehandlung einer Katalysator-Vorstufe mit einem Kohlenmonoxid und Wasserstoff enthaltenden Gasgemisch durch. Die erfindungsgemäße asymmetrische Hydrierung führt man in Gegenwart von dem Reaktionsgemisch zusätzlich zugeführtem Kohlenmonoxid durchführt.

Das erfindungsgemäße Verfahren ist dadurch gekennzeichnet ist, dass man die genannte Vorbehandlung der Katalysator-Vorstufe mit einem Gasgemisch umfassend 30 bis 70 Vol.-% Kohlenmonoxid, 30 bis 70 Vol-% Wasserstoff und 0 bis 5 Vol.-% weiterer Gase, wobei sich die genannten Volumenanteile zu 100 Vol.-% ergänzen, bei einem Druck von 50 bis 90 bar durchführt, von dem so erhaltenen Katalysator vor Einsatz in der asymmetrischen Hydrierung überschüssiges Kohlenmonoxid abtrennt und die asymmetrische Hydrierung in Gegenwart von Wasserstoff mit einem Kohlenmonoxidgehalt von 100 bis 1200 ppm durchführt.

Die erfindungsgemäß einzusetzenden, im Reaktionsgemisch löslichen Übergangsmetall-Katalysatoren weisen zumindest in einer im Katalysezyklus durchlaufenen Form oder in einer dem eigentlichen Katalysezyklus vorgeschalteten Vorform mindestens einen CO-Liganden auf, wobei es unerheblich ist, ob diese mindestens einen CO-Liganden aufweisende Katalysatorform die tatsächliche katalytisch aktive Katalysatorform darstellt. Im Rahmen des erfindungsgemäßen Verfahrens stabilisiert man die mindestens einen CO-Liganden aufweisende Katalysatorform durch dem Reaktionsgemisch zusätzlich zugeführtes Kohlenmonoxid in vorteilhafter Weise.

Das erfindungsgemäße Verfahren eignet sich zur Herstellung optisch aktiver Carbonylverbindungen der Formel (I) 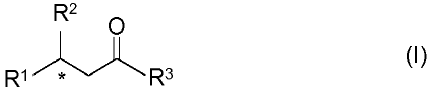 wobei die Reste
- R¹, R²: jeweils für einen unverzweigten, verzweigten oder cyclischen Alkylrest mit 1 bis 25 Kohlenstoffatomen, der gegebenenfalls eine oder mehrere, in der Regel 1 bis etwa 5, bevorzugt 1 bis 3, besonders bevorzugt 1 oder 2, ethylenische Doppelbindungen und/oder einen oder mehrere, in der Regel 1 bis etwa 5, bevorzugt 1 bis 3, besonders bevozugt 1 oder 2 gleiche oder verschiedene Substituenten ausgewählt aus der Gruppe der Substituenten OR⁴, NR⁵R⁶, Halogen, C₆-C₁₀-Aryl und C₃-C₉-Hetaryl tragen kann und der gemeinsam mit R³ einen 5 bis 25-gliedrigen Ring bilden kann, stehen, mit der Maßgabe, dass R¹ und R² verschieden sind,
- R³: für Wasserstoff oder einen unverzweigten, verzweigten oder cyclischen Alkylrest mit 1 bis 25 Kohlenstoffatomen, der gegebenenfalls eine oder mehrere, in der Regel 1 bis etwa 5, bevorzugt 1 bis 3, besonders bevorzugt 1 oder 2, ethylenische Doppelbindungen und/oder einen oder mehrere, in der Regel 1 bis etwa 5, bevorzugt 1 bis 3, besonders bevorzugt 1 oder 2, gleiche oder verschiedene Substituenten ausgewählt aus der Gruppe der Substituenten OR⁴, NR⁵R⁶, Halogen, C₆-C₁₀-Aryl und C₃-C₉-Hetaryl tragen kann, oder für OR⁷ oder NR⁸R⁹ steht,
wobei
- R⁴, R⁵, R⁶: jeweils unabhängig voneinander Wasserstoff, C₁-C₆-Alkyl, C₆-C₁₀-Aryl, C₇-C₁₂-Aralkyl oder C₇-C₁₂-Alkylaryl bedeuten und
- R⁵ und R⁶: gemeinsam auch eine Alkylenkette mit 2 bis 5 Kohlenstoffatomen, die durch N oder O unterbrochen sein kann, bedeuten können und
- R⁷: für einen unverzweigten, verzweigten oder cyclischen Alkylrest mit 1 bis 25 Kohlenstoffatomen, der gegebenenfalls eine oder mehrere ethylenische Doppelbindungen und eine oder mehrere gleiche oder verschiedene Substituenten ausgewählt aus der Gruppe der Substituenten OR⁴, NR⁵R⁶, Halogen, C₆-C₁₀-Aryl und C₃-C₉-Hetaryl tragen kann, stehen und gemeinsam mit R¹ oder R² einen 5 bis 25-gliedrigen Ring bilden können und
- R⁸: für einen unverzweigten, verzweigten oder cyclischen Alkylrest mit 1 bis 25 Kohlenstoffatomen, der gegebenenfalls eine oder mehrere, bevorzugt 1 bis 3, besonders bevorzugt 1 oder 2, ethylenische Doppelbindungen und eine oder mehrere, bevorzugt 1 bis 3, besonders bevorzugt 1 oder 2, gleiche oder verschiedene Substituenten ausgewählt aus der Gruppe der Substituenten OR⁴, NR⁵R⁶, Halogen, C₆-C₁₀-Aryl und C₃-C₉-Hetaryl tragen kann, stehen und gemeinsam mit R¹, R² oder R⁹ einen 5 bis 25-gliedrigen Ring bilden kann und
- R⁹: für Wasserstoff, C₁-C₆-Alkyl, C₆-C₁₀-Aryl, C₇-C₁₂-Aralkyl oder C₇-C₁₂-Alkylaryl steht oder zusammen mit R⁸ einen 5 bis 25-gliedrigen Ring bilden kann und
- *: ein asymmetrisches Kohlenstoffatom bezeichnet,
durch asymmetrische Hydrierung a,β-ungesättigter Aldehyde oder Ketone der Formel (II) 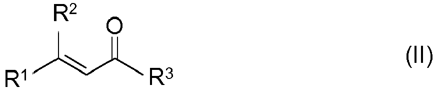 wobei die Reste R¹ bis R³ die oben angegebene Bedeutung besitzen.

Beispielhaft seien für die genannten Substituenten bzw. Reste folgende Bedeutungen im Rahmen der vorliegenden Erfindung angegeben:
C₁-C₆-Alkyl steht beispielsweise für Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl oder 1-Ethyl-2-methylpropyl.

C₆-C₁₀-Aryl bedeutet beispielsweise Phenyl oder Naphtyl.

C₇-C₁₂-Aralkyl bedeutet beispielsweise Phenylmethyl, 1-Phenylethyl 2-Phenylethyl, 1-Phenylpropyl, 2-Phenylpropyl oder 3-Phenylpropyl.

C₃-C₉-Hetaryl steht beispielsweise für 2-Furyl, 3-Furyl, 2-Pyrroyl, 3-Pyrroyl, 2-Thienyl, 3-Thienyl, 2-Pyridyl, 3-Pyridyl, 4-Pyridyl, 2-Indolyl, 3-Indolyl, 4-Indolyl, 5-Indolyl, 6-Indolyl, 7-Indolyl.

C₇-C₁₂-Alkylaryl steht beispielsweise für 1-Methylphenyl, 2-Methylphenyl, 3-Methylphenyl, 1-Ethylphenyl, 2-Ethylphenyl, 3-Ethylphenyl, 1-Propylphenyl, 2-Propylphenyl, 3-Propylphenyl, 1-iso-Propylphenyl, 2-iso-Propylphenyl, 3-iso-Propylphenyl, 1-Butylphenyl, 2-Butylphenyl, 3-Butylphenyl, 1-iso-Butylphenyl, 2-iso-Butylphenyl, 3-iso-Butylphenyl, 1-sec-Butylphenyl, 2-sec-Butylphenyl, 3-sec-Butylphenyl, 1-tert-Butylphenyl, 2-tert-Butylphenyl, 3-tert-Butylphenyl, 1-(1-pentenyl)phenyl, 2-(1-Pentenyl)phenyl, 3-(1-Pentenyl)phenyl, 1-(2-Pentenyl)phenyl, 2-(2-Pentenyl)phenyl, 3-(2-Pentenyl)phenyl, 1-(3-Pentenyl)phenyl, 2-(3-Pentenyl)phenyl, 3-(3-Pentenyl)phenyl, 1-(1-(2-Methylbutyl))phenyl, 2-(1-(2-Methylbutyl))phenyl, 3-(1-(2-Methylbutyl))phenyl, 1-(2-(2-Methylbutyl))phenyl, 2-(2-(2-Methylbutyl))phenyl, 3-(2-(2-Methylbutyl))phenyl, 1-(3-(2-Methylbutyl))phenyl, 2-(3-(2-Methylbutyl))phenyl, 3-(3-(2-Methylbutyl))phenyl, 1-(4-(2-Methylbutyl))phenyl, 2-(4-(2-Methylbutyl))phenyl, 3-(4-(2-Methylbutyl))phenyl, 1-(1-(2,2-Dimethylpropyl))phenyl, 2-(1-(2,2-Dimethylpropyl))phenyl, 3-(1-(2,2-Dimethylpropyl))phenyl, 1-(1-hexenyl)phenyl, 2-(1-Hexenyl)phenyl, 3-(1-Hexenyl)phenyl, 1-(2-Hexenyl)phenyl, 2-(2-Hexenyl)phenyl, 3-(2-Hexenyl)phenyl, 1-(3-Hexenyl)phenyl, 2-(3-Hexenyl)phenyl, 3-(3-Hexenyl)phenyl, 1-(1-(2-Methylpentenyl))phenyl, 2-(1-(2-Methylpentenyl))phenyl, 3-(1-(2-Methylpentenyl))phenyl, 1-(2-(2-Methylpentenyl))phenyl, 2-(2-(2-Methylpentenyl))phenyl, 3-(2-(2-Methylpentenyl))phenyl, 1-(3-(2-Methylpentenyl))phenyl, 2-(3-(2-Methylpentenyl))phenyl, 3-(3-(2-Methylpentenyl))phenyl, 1-(4-(2-Methylpentenyl))phenyl, 2-(4-(2-Methylpentenyl))phenyl, 3-(4-(2-Methylpentenyl))phenyl, 1-(5-(2-Methylpentenyl))phenyl, 2-(5-(2-Methylpentenyl))phenyl, 3-(5-(2-Methylpentenyl))phenyl, 1-(1-(2,2-Dimethylbutenyl))phenyl, 2-(1-(2,2-Dimethylbutenyl))phenyl, 3-(1-(2,2-Dimethylbutenyl))phenyl, 1-(3-(2,2-Dimethylbutenyl))phenyl, 2-(3-(2,2-Dimethylbutenyl))phenyl, 3-(3-(2,2-Dimethylbutenyl))phenyl, 1-(4-(2,2-Dimethylbutenyl))phenyl, 2-(4-(2,2-Dimethylbutenyl))phenyl, 3-(4-(2,2-Dimethylbutenyl))phenyl.

Unter Halogen ist im Rahmen der vorliegenden Erfindung Fluor, Chlor, Brom oder Iod, bevorzugt Fluor, Chlor oder Brom zu verstehen.

Das erfindungsgemäße Verfahren eignet sich demgemäss beispielsweise zur Herstellung der folgenden, beispielhaft genannten Verbindungen der Formeln (I-1) bis (I-25) in optisch aktiver Form:

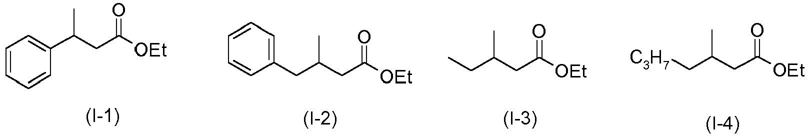

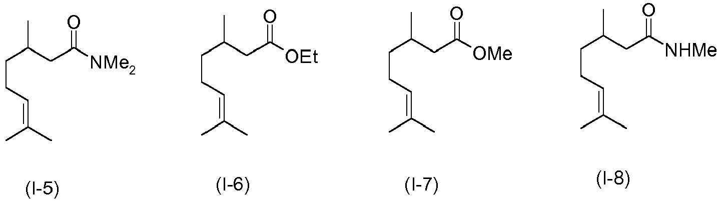

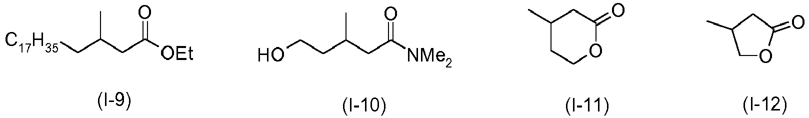

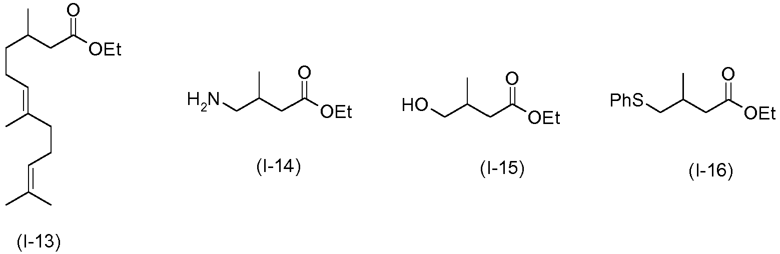

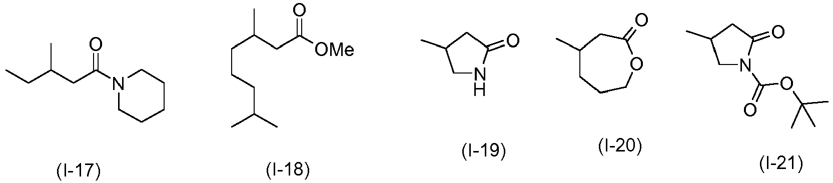

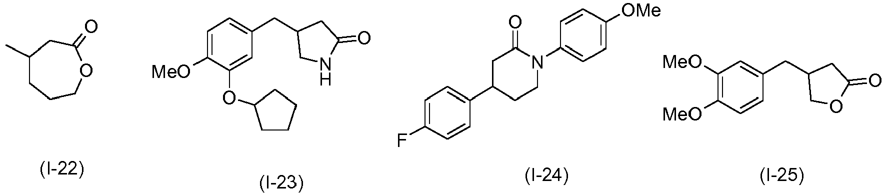

Das erfindungsgemäße Verfahren eignet sich insbesondere zur Herstellung optisch aktiver Verbindungen der Formel (I') 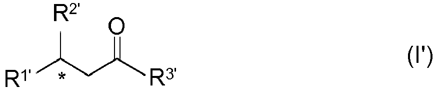 in der
- R^{1'}, R^{2'}: die gleichen Bedeutungen haben können wie vorstehend R¹ und R² und
- R^{3'}: für Wasserstoff oder einen unverzweigten, verzweigten oder cyclischen Alkylrest mit 1 bis 25 Kohlenstoffatomen, der gegebenenfalls eine oder mehrere, in der Regel 1 bis etwa 5, bevorzugt 1 bis 3, besonders bevorzugt 1 oder 2, ethylenische Doppelbindungen und/oder einen oder mehrere, in der Regel 1 bis etwa 5, bevorzugt 1 bis 3, besonders bevorzugt 1 oder 2, gleiche oder verschiedene Substituenten ausgewählt aus der Gruppe der Substituenten OR⁴, NR⁵R⁶, Halogen, C₆-C₁₀-Aryl und C₃-C₉-Hetaryl tragen kann und gemeinsam mit R^{1'} oder R^{2'} einen 5 bis 25-gliedrigen Ring bilden kann, steht,
wobei R⁴, R⁵ und R⁶ die vorstehend bezeichneten Bedeutungen besitzen können,
durch asymmetrische Hydrierung a,β-ungesättigter Aldehyde oder Ketone der Formel (II') 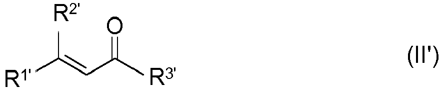 wobei die Reste R^{1'} bis R^{3'} die oben angegebene Bedeutung besitzen.

Bevorzugt eignet sich das erfindungsgemäße Verfahren zur Herstellung optisch aktiver Aldehyde der Formel (III), die in β-Position zur Carbonylgruppe eine Methylgruppe aufweisen 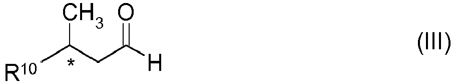 wobei
- R¹⁰: für einen unverzweigten oder verzweigten Alkylrest mit 2 bis 25 Kohlenstoffatomen, der gegebenenfalls 1 bis 5, bevorzugt 1 bis 3, besonders bevorzugt 1 oder 2, ethylenische Doppelbindungen aufweisen kann, steht und
- *: ein asymmetrisches Kohlenstoffatom bezeichnet,
durch asymmetrische Hydrierung a,β-ungesättigter Aldehyde der Formel (IV) oder (V) 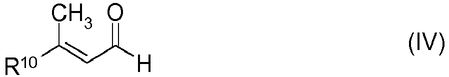 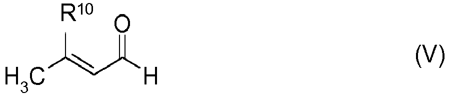 wobei der Rest R¹⁰ die oben angegebene Bedeutung besitzt.

Als Beispiele für erfindungsgemäß in optisch aktiver Form herstellbare Aldehyde oder Ketone der Formeln (I') bzw. (III) seien die folgenden Verbindungen genannt:

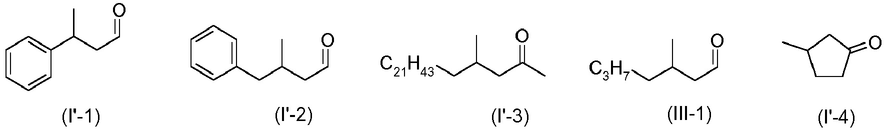

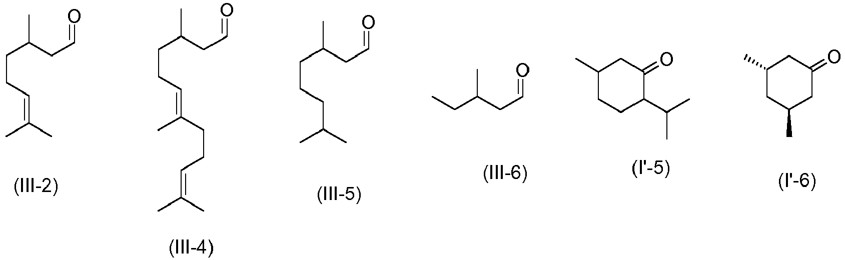

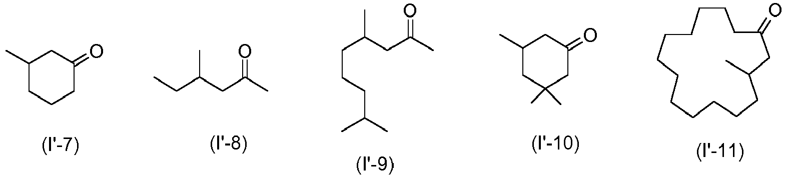

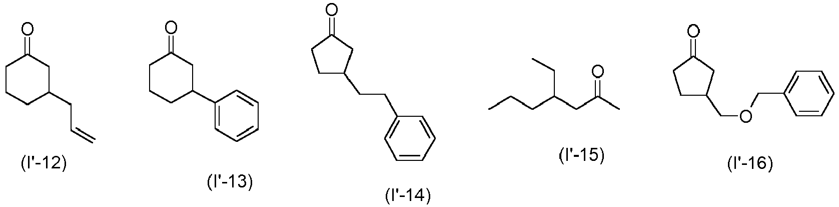

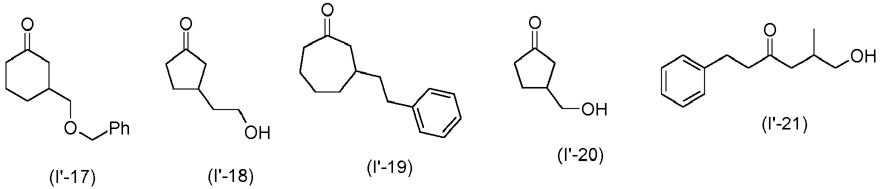

Die Aldehyde der Formel (III) sind erfindungsgemäß durch asymmetrische, d.h. enantioselektive Hydrierung der entsprechenden α,β-ungesättigten Aldehyde der Formeln (IV) oder (V) zugänglich. Die Verbindungen der Formeln (IV) und (V) stellen E/Z-Doppelbindungsisomere zueinander dar. Prinzipiell sind die optisch aktiven Aldehyde der Formel (III) ausgehend von beiden Doppelbindungsisomeren der Formeln (IV) und (V) zugänglich. Je nach Wahl der enantiomeren Form des Katalysators , d.h. je nach Wahl des (+)- bzw. (-)-Enantiomeren des Katalysators bzw. des (+)- bzw. (-)-Enantiomeren des eingesetzten chiralen Liganden, erhält man in erfindungsgemäßer Weise aus dem eingesetzten E- oder Z-Doppelbindungsisomeren bevorzugt eines der Enantiomere des optisch aktiven Aldehyds. Gleiches gilt für die vorstehend genannten Substrat- bzw. Produktklassen. Prinzipiell lassen sich auch Gemische der beiden Doppelbindungsisomere in erfindungsgemäßer Weise umsetzen. Man erhält auf diese Weise Gemische der beiden Enantiomere der gewünschten Zielverbindung.

Besonders bevorzugt eignet sich das erfindungsgemäße Verfahren zur Herstellung von optisch aktivem Citronellal der Formel (VI) 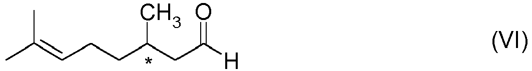 durch asymmetrische Hydrierung von Neral der Formel (VII) oder Geranial der Formel (VIII)

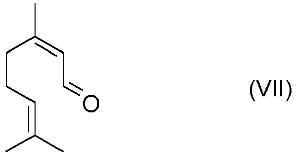

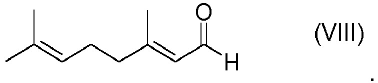

Auch Gemische von Geranial und Neral lassen sich in erfindungsgemäßer Weise umsetzen, wobei man, wie vorstehend beschrieben Gemische von D- bzw. L-Citronellal enthält, die optisch aktiv sind, falls die beiden Enantiomere nicht zu gleichen Teilen darin vorliegen.

Im Rahmen des erfindungsgemäßen Verfahrens insbesondere bevorzugt ist die erfindungsgemäße Herstellung D-Citronellal durch asymmetrische Hydrierung von Neral bzw. Geranial.

Das erfindungsgemäße Herstellverfahren wird in Gegenwart eines im Reaktionsgemisch löslichen, optisch aktiven Übergangsmetall-Katalysators, der mindestens einen Kohlenmonoxid-Liganden aufweist, durchgeführt.

Derartige Katalysatoren sind beispielsweise erhältlich durch Reaktion mindestens einer geeigneten, im Reaktionsgemisch löslichen Übergangsmetall-Verbindung mit einem optisch aktiven Liganden, der mindestens ein Phosphor- und/oder Arsenatom aufweist.

Bevorzugte Übergangsmetall-Verbindungen sind solche der Metalle der VIII. Nebengruppe des Periodensystems der Elemente, insbesondere Ru, Rh, Pd, Ir und Pt. Erfindungsgemäß besonders bevorzugte Übergangsmetalle der VIII. Nebengruppe des Periodensystems sind Rh und Ir.

Geeignete Verbindungen der genannten Übergangsmetalle sind insbesondere solche, die im gewählten Reaktionsmedium löslich sind, wie beispielsweise Salze oder Komplexverbindungen mit geeigneten Liganden wie z.B. Carbonyl, Acetylacetonat, Hydroxy, Cyclooctadien, Norbornadien, Cycloocten, Methoxy, Acetyl oder sonstige aliphatische oder aromatische Carboxylate. Im Rahmen des erfindungsgemäßen Verfahrens bevorzugte Übergangsmetallverbindungen sind Rh(I)- und Rh(III)- sowie Rh(0)-Verbindungen, Ir(I)-, Ir(III), Ir(IV)- sowie Ir(0)-Verbindungen, Ru(II), Ru(III), Ru(IV)-sowie Ru(0)-verbindungen, Pd(II)-, Pd(IV)- sowie Pd(0)-Verbindungen und Pt(II)-, Pt(IV)-sowie Pt(0)-Verbindungen. Bevorzugt sind solche Übergangsmetall-Verbindungen, die bereits mindestens einen CO-Liganden aufweisen. Daneben lassen sich auch Übergangsmetall-Verbindungen, die keinen CO-Liganden aufweisen, im Rahmen des erfindungsgemäßen Verfahrens als Ausgangsverbindung zur Herstellung der erfindungsgemäß einzusetzenden Katalysatoren verwenden. Diese werden unter den Bedingungen der erfindungsgemäß wahlweise durchzuführenden Präformierung bzw. der erfindungsgemäßen Hydrierbedingungen unter Zusatz von Kohlenmonoxid in die gewünschten Katalysatoren überführt.

Beispiele für erfindungsgemäß einsetzbare Übergangsmetall-Verbindungen sind: RhCl₃, Rh₂(OAc)₄, [Rh(cod)Cl]₂, Rh(CO)₂acac, [Rh(cod)OH]₂, [Rh(cod)OMe]₂, Rh₄(CO)₁₂, Rh₆(CO)₁₆ bzw. Ir₄(CO)₁₂, [Ir(cod)Cl]₂, wobei "acac" für einen Acetylacetonat- und "cod" für einen Cyclooctadien-Liganden steht.

Die genannten und weitere geeignete Übergangsmetallverbindungen und -komplexe sind bekannt und in der Literatur hinreichend beschrieben oder können vom Fachmann analog zu den bereits bekannten Verbindungen hergestellt werden.

Die genannten Übergangsmetallverbindungen setzt man erfindungsgemäß üblicherweise in einer Menge von etwa 0,01 bis etwa 1 mol-%, bevorzugt von etwa 0,05 bis etwa 0,5 mol-%, insbesondere von etwa 0,02 bis etwa 0,2 mol-% (bezogen auf die enthaltenen Übergangsmetallatome) im Verhältnis zur Menge an zu hydrierendem Substrat ein.

Bei unter kontinuierlichen Bedingungen durchgeführten Umsetzungen wählt man das Verhältnis von eingesetzter Menge an Übergangsmetallverbindung als Vorläufer des erfindungsgemäßen homogenen Katalysators zur Menge an zu hydrierendem Substrat vorteilhaft so, dass eine Katalysatorkonzentration im Bereich von etwa 100 ppm bis 10000 ppm, insbesondere im Bereich von etwa 200 ppm bis 5000 ppm eingehalten wird.

Die genannten Übergangsmetallverbindungen werden erfindungsgemäß mit einer weiteren Verbindung in Kontakt gebracht, die optisch aktiv, bevorzugt im wesentlichen enantiomerenrein ist (d.h. einen Enantiomerenüberschuss von mindestens etwa 99% aufweist) und mindestens ein Phosphor und/oder Arsenatom, bevorzugt mindestens ein Phosphoratom aufweist. Diese als chiraler Ligand zu bezeichnende Verbindung bildet im Reaktionsgemisch bzw. im Präformierungsgemisch mit der eingesetzten Übergangsmetall-Verbindung den erfindungsgemäß einzusetzenden Übergangsmetall-Katalysator.

Insbesondere bevorzugt sind solche chiralen Liganden die zwei Phosphoratome aufweisen und mit dem eingesetzten Übergangsmetall Chelatkomplexe bilden.

Als chirale Liganden eignen sich im Rahmen der vorliegenden Erfindung solche Verbindungen, wie sie beispielsweise in: I. Ojima (Hrsg.), Catalytic Asymmetrie Synthesis, Wiley-VCh, 2. Auflage, 2000 oder in E. N. Jacobsen, A. Pfaltz, H. Yamamoto (Hrsg.), Comprehensive Asymmetric Catalysis, 2000, Springer oder in W. Tang, X. Zhang, Chem. Rev. 2003, 103, 3029-3069 beschrieben sind.

Beispielhaft seien die folgenden Verbindungen als erfindungsgemäß bevorzugt einsetzbare chirale Liganden angeführt:

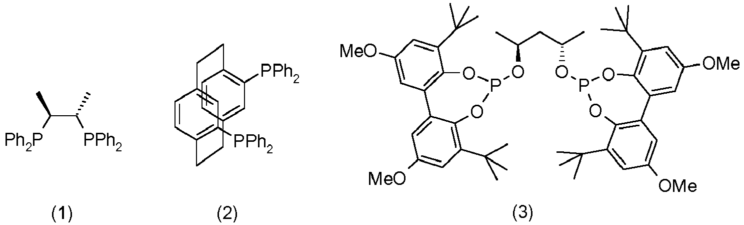

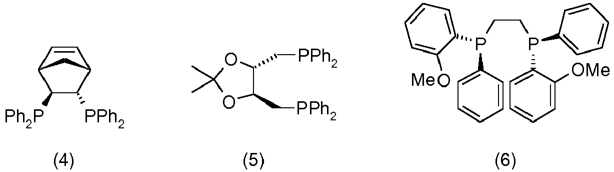

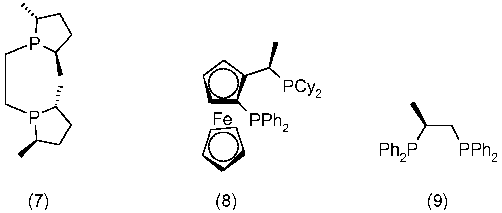

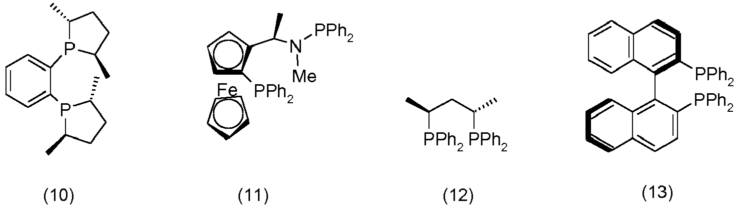

Weiterhin seien als erfindungsgemäß einsetzbare chirale Liganden beispielhaft die folgenden Verbindungen angeführt:

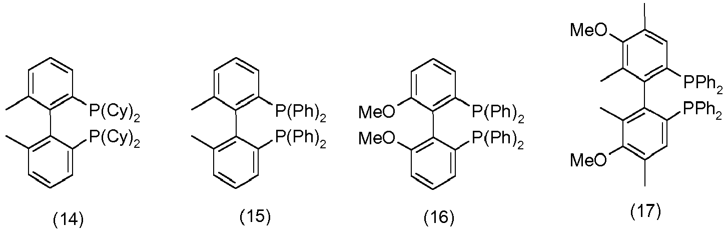

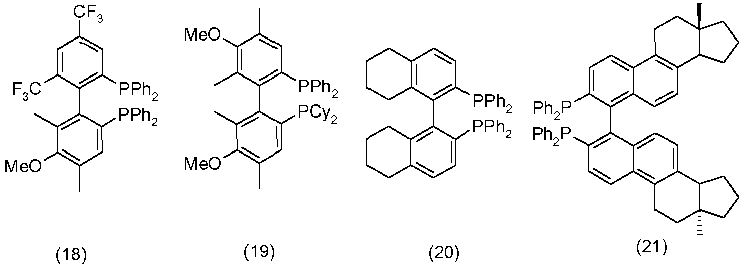

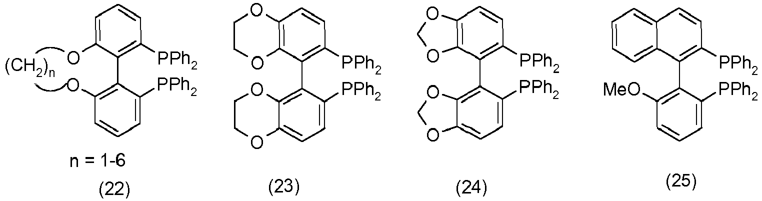

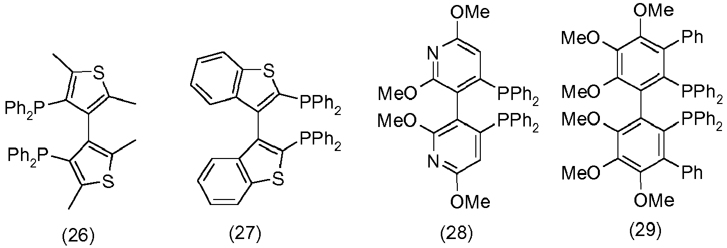

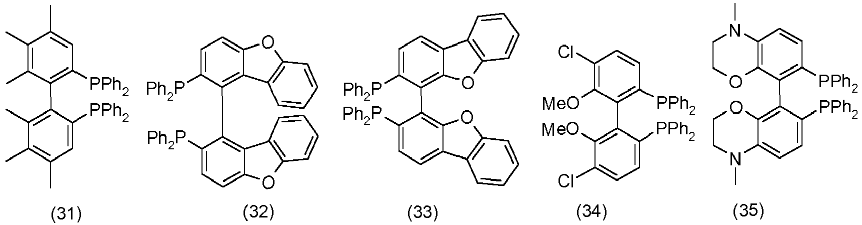

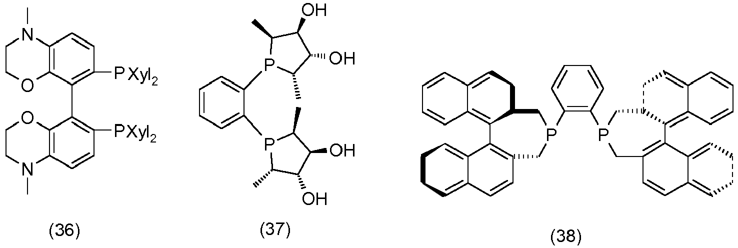

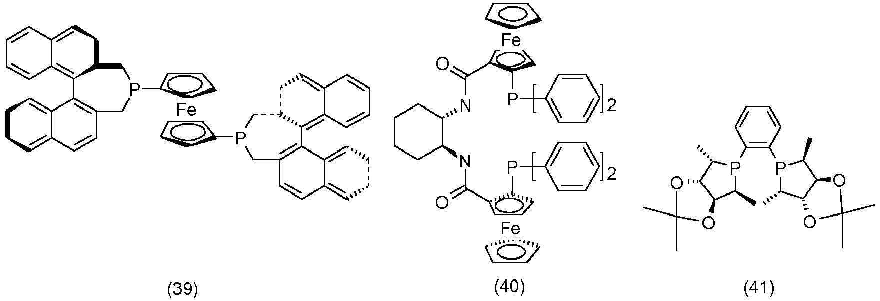

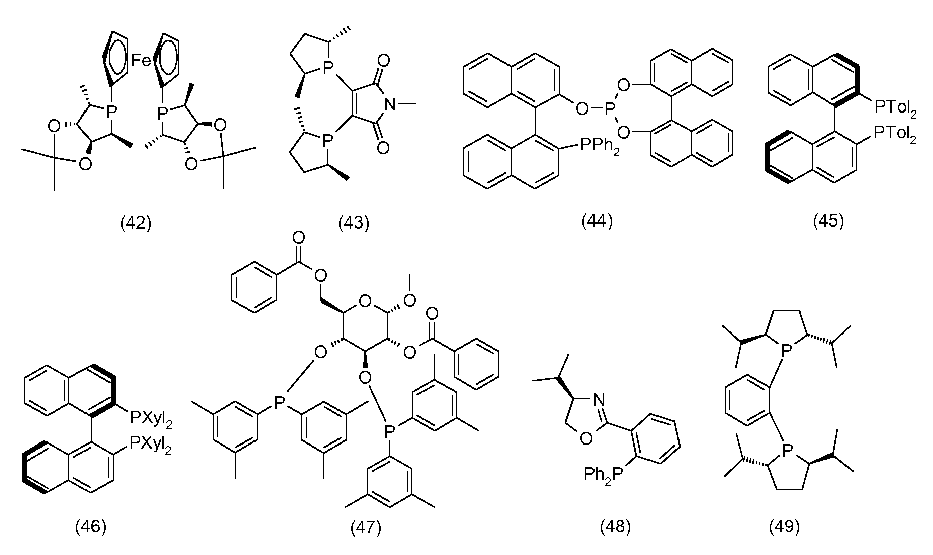

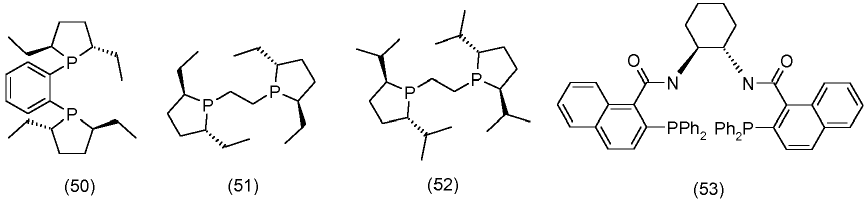

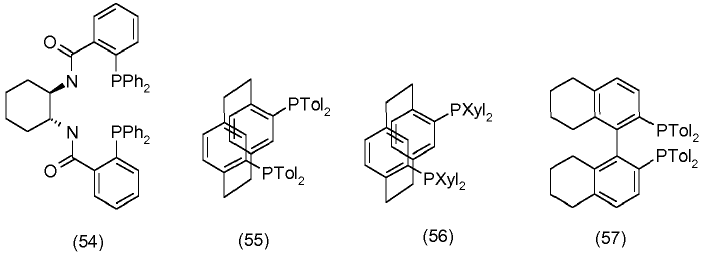

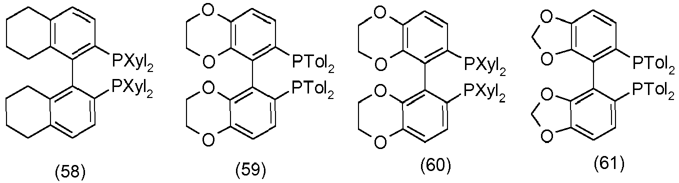

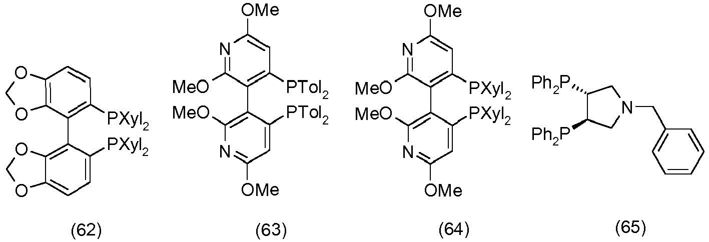

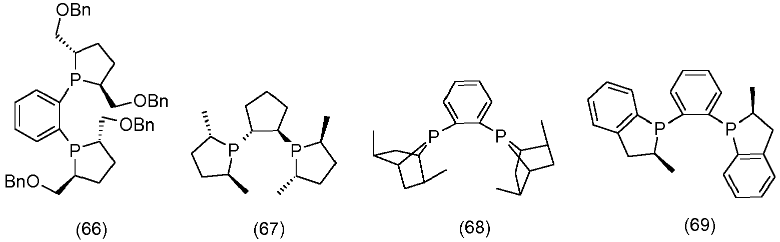

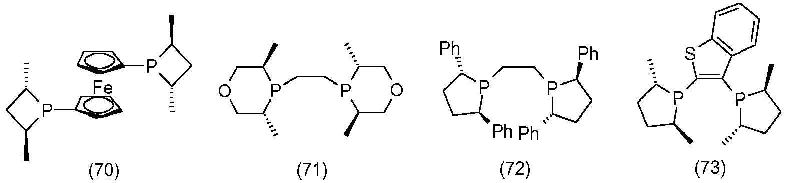

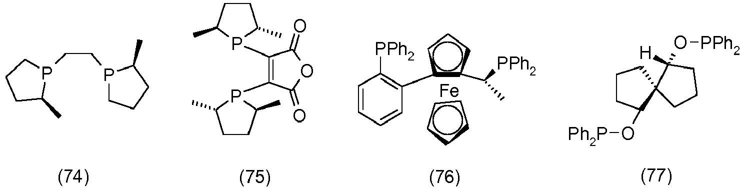

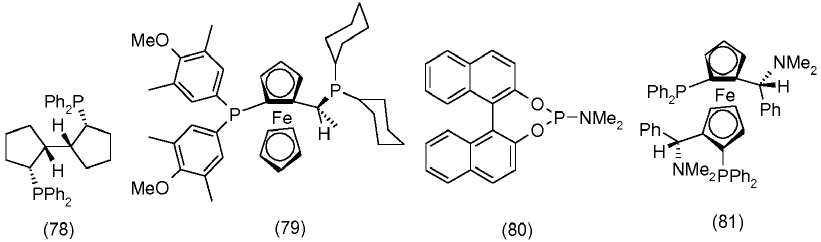

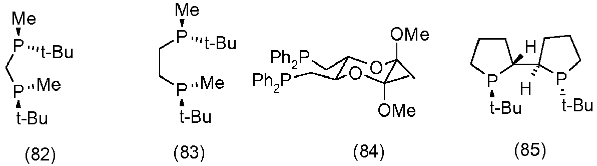

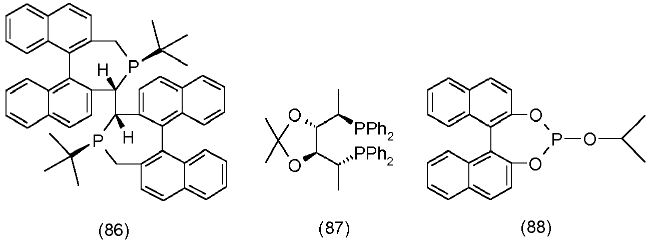

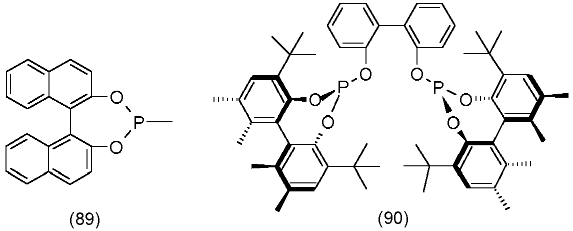

Dabei ist unter "Ph" Phenyl, "Cy" Cyclohexyl, "Xyl" Xylyl, "Tol" p-Tolyl und "Bn" Benzyl zu verstehen.

Erfindungsgemäß besonders bevorzugte Liganden sind die der Strukturformeln (1) bis (13) sowie (37), (38), (41), (43), (49), (50), (51), (52), (65), (66), (67), (68), (69), (71), (72), (73), (74), (75), (83), (84), (85), (86), (87).

Insbesondere bevorzugte Liganden sind solche der allgemeinen Formeln (IX) bis (XI) 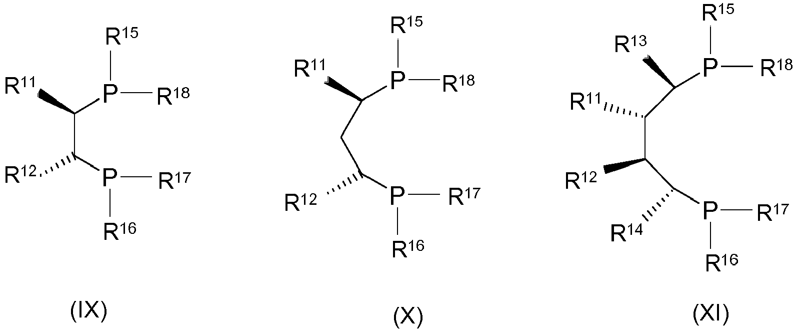 in denen
- R¹¹, R¹²:: jeweils unabhängig voneinander für einen unverzweigten, verzweigten oder cyclischen Alkylrest mit 1 bis 20 Kohlenstoffatomen, der gegebenenfalls eine oder mehrere, in der Regel 1 bis etwa 4, ethylenische Doppelbindungen und/oder einen oder mehrere, in der Regel 1 bis etwa 4, gleiche oder verschiedene Substituenten ausgewählt aus der Gruppe der Substituenten OR¹⁹, NR²⁰R²¹, Halogen, C₆-C₁₀-Aryl und C₃-C₉-Hetaryl tragen kann und R¹¹ und R¹² gemeinsam einen 4- bis 20-gliedrigen Ring bilden können, der eines oder mehrere, in der Regel 1 oder 2 O-Atome beinhalten kann, stehen und
- R¹³, R¹⁴:: jeweils unabhängig voneinander Wasserstoff oder geradkettiges oder verzweigtes C₁- bis C₄-Alkyl bedeuten und
- R¹⁵, R¹⁶, R¹⁷, R¹⁸:: jeweils für C₆- bis C₁₀-Aryl, das gegebenenfalls einen oder mehrere, in der Regel 1 bis 8, bevorzugt 1 bis 4 Substituenten ausgewählt aus der Gruppe der Substituenten C₁- bis C₄-Alkyl, C₆- bis C₁₀-Aryl, C₁-bis C₄-Alkoxy und Amino tragen kann, stehen und
- R¹⁹, R²⁰, R²¹:: jeweils unabhängig voneinander Wasserstoff, C₁-C₄-Alkyl, C₆-C₁₀-Aryl, C₇-C₁₂-Aralkyl oder C₇-C₁₂-Alkylaryl bedeuten, wobei
- R²⁰, R²¹:: gemeinsam auch eine Alkylenkette mit 2 bis 5 Kohlenstoffatomen, die durch N oder O unterbrochen sein kann, bedeuten können.

Im Rahmen des erfindungsgemäßen Verfahrens insbesondere bevorzugte Liganden sind solche der allgemeinen Formel (IX), insbesondere die im folgenden als "Chiraphos" bezeichneten Verbindungen der Formel (1).

Die gewählten chiralen Liganden können erfindungsgemäß jeweils in Form ihrer beiden Enantiomere eingesetzt werden. Ein erfindungsgemäß ganz besonders bevorzugter Ligand ist (R,R)-Chiraphos (ent-(1)).

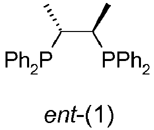

Bei Einsatz von chiralen Liganden mit zwei Phosphoratomen setzt man diese vorteilhaft in einer Menge von etwa 1 bis etwa 10 mol, bevorzugt etwa 1 bis etwa 4 mol, ganz besonders bevorzugt 1 bis 2 mol pro mol-Äquivalent an Metall, das in der eingesetzten Übergangsmetall-Verbindung enthalten ist, ein.

Aus der gewählten Übergangsmetall-Verbindung und dem gewählten chiralen Liganden können die eigentlichen, mindestens einen Kohlenmonoxid-Liganden enthaltenden Präkatalysatoren durch Zusammenbringen und anschließende, wie nachfolgend beschriebene Vorbehandlung mit einer Mischung aus Wasserstoff und Kohlenmonoxid erhalten werden.

Zur Herstellung des jeweils einzusetzenden optisch aktiven, mindestens einen Kohlenmonoxid-Liganden aufweisenden Katalysators behandelt man gemäß Schritt a) des erfindungsgemäßen Verfahrens eine Katalysator-Vorstufe mit einem Gasgemisch umfassend 30 bis 70 Vol.-% Kohlenmonoxid, 30 bis 70 Vol-% Wasserstoff und 0 bis 5 Vol.-% weiterer Gase, wobei sich die genannten Volumenanteile zu 100 Vol.-% ergänzen, bei einem Druck von 50 bis 90 bar vor. Diese Vorbehandlung wird im folgenden wie im Rahmen der gesamten vorliegenden Erfindung auch als Präformierung bezeichnet.

Unter dem Begriff Katalysator-Vorstufe sind dabei solche Verbindungen zu verstehen, die erhältlich sind durch Inkontaktbringen bzw. durch Reaktion mindestens einer wie vorstehend genannten, im Reaktionsgemisch löslichen Übergangsmetall-Verbindung mit einem wie vorstehend genannten optisch aktiven Liganden, der mindestens ein Phosphor- und/oder Arsenatom aufweist.

Zur Durchführung der Präformierung löst man üblicherweise die gewählte Übergangsmetall-Verbindung und den gewählten chiralen Liganden und gewünschtenfalls das asymmetrisch zu hydrierende Substrat in einem geeigneten, unter den Reaktionsbedingungen inerten Lösungsmittel bzw. Lösungsmedium wie beispielsweise Ether, Tetrahydrofuran, Toluol, Chlorbenzol, Octadecanol, Biphenylether, Texanol, Marlotherm^{®}, Oxoöl 9N (Hydroformylierungsprodukte aus isomeren Octenen, BASF Aktiengesellschaft) und dergleichen mehr. Als Lösungsmedium können auch das umzusetzende Substrat, das Produkt oder bei der Umsetzung eventuell anfallende hochsiedende Nebenprodukte dienen. Der resultierenden Lösung wird, vorteilhafterweise in einem geeigneten Druckreaktor bzw. Autoklaven, bei einem Druck im Bereich von 50 bis 90 bar (jeweils absolut), ein wie vorstehend beschriebenes, Wasserstoff und Kohlenmonoxid enthaltendes Gasgemisch aufgepresst.

Im Rahmen des erfindungsgemäßen Verfahrens führt man die Präformierung gemäß Schritt a) mit einem Gasgemisch umfassend 30 bis 70 Vol.-% Kohlenmonoxid, 30 bis 70 Vol-% Wasserstoff und 0 bis 5 Vol.-% weiterer Gase, wobei sich die genannten Volumenanteile zu 100 Vol.-% ergänzen, ganz besonders bevorzugt mit einem Gasgemisch umfassend 30 bis 60 Vol.-% Kohlenmonoxid, 40 bis 70 Vol.-% Wasserstoff und 0 bis 5 Vol.-% weiterer Gase und insbesondere bevorzugt mit einem Gasgemisch umfassend 40 bis 60 Vol.-% Kohlenmonoxid, 40 bis 60 Vol.-% Wasserstoff und 0 bis 5 Vol.-% weiterer Gase, wobei sich die genannten Volumenanteile jeweils zu 100 Vol.-% ergänzen, durch.

Ein zur Präformierung insbesondere bevorzugtes Gasgemisch ist sogenanntes Synthesegas, das üblicherweise zu etwa 35 bis 55 Vol.-% aus Kohlenmonoxid und etwa 45 bis 65 Vol.-% Wasserstoff sowie gegebenenfalls Spuren weiterer Gase besteht.

Die erfindungsgemäße Präformierung des Katalysators wird üblicherweise bei Temperaturen von etwa 25°C bis etwa 100°C, bevorzugt bei etwa 40°C bis etwa 80°C, besonders bevorzugt bei 50-70°C durchgeführt. Führt man die Präformierung in Gegenwart des asymmetrisch zu hydrierenden Substrats durch, wählt man die Temperatur mit Vorteil so, dass es nicht in störendem Ausmaß zu einer Isomerisierung der zu hydrierenden Doppelbindung kommt. Die Präformierung ist üblicherweise nach etwa 1 bis etwa 24 h, oft nach etwa 1 bis etwa 12 h abgeschlossen.

Im Anschluss an die Präformierung des einzusetzenden Übergangsmetall-Katalysators bzw. dessen Vorstufe trennt man gemäß Schritt b) des erfindungsgemäßen Verfahrens von dem durch Präformierung bzw. Vorbehandlung mit dem genannten Gasgemisch erhaltenen Katalysator vor Einsatz in der asymmetrischen Hydrierung überschüssiges Kohlenmonoxid ab.

Unter dem Begriff überschüssiges Kohlenmonoxid ist dabei solches Kohlenmonoxid zu verstehen, das in dem gemäß Schritt a) durch Präformierung erhaltenen Reaktionsgemisch in gasförmiger oder gelöster Form enthalten ist und nicht an den Übergangsmetall-Katalysator bzw. dessen Vorstufe gebunden ist. Demgemäss entfernt man das überschüssige, nicht an den Katalysator gebundene Kohlenmonoxid zumindest weitgehend, d.h. in einem Ausmaß, dass sich eventuelle Restmengen gelösten Kohlenmonoxids in der folgenden Hydrierung nicht störend bemerkbar machen. Dies ist üblicherweise gewährleistet, wenn etwa 90%, bevorzugt etwa 95% oder mehr des zur Präformierung eingesetzten Kohlenmonoxids gemäß Schritt b) des erfindungsgemä-ßen Verfahrens abgetrennt werden. Bevorzugt entfernt man gemäß Schritt b) des erfindungsgemäßen Verfahrens überschüssiges Kohlenmonoxid vollständig von dem durch Präformierung erhaltenen Katalysator.

Die Abtrennung des überschüssigen Kohlenmonoxids vom gemäß Schritt a) erhaltenen Katalysator bzw. vom den Katalysator enthaltenden Reaktionsgemisch gemäß Schritt b) des erfindungsgemäßen Verfahrens erfolgt, indem man den Katalysator bzw. das durch Präformierung gemäß Schritt a) erhaltene, den Katalysator enthaltende Gemisch entspannt auf einen Druck im Bereich von 1 bis 3 bar, ganz besonders bevorzugt auf einen Druck im Bereich von etwa 1 bis etwa 2 bar, insbesondere bevorzugt auf Normaldruck, so dass gasförmiges, nicht gebundenes Kohlenmonoxid aus dem Produkt der Präformierung entweicht.

Die vorstehend genannte Entspannung des präformierten Katalysators kann beispielsweise unter Einsatz eines Hochdruckabscheiders, wie er dem Fachmann an sich bekannt ist, erfolgen. Derartige Abscheider, bei denen die Flüssigkeit in der kontinuierlichen Phase ist, sind beispielsweise beschrieben in: Perry's Chemical Engineers' Handbook, 1997, 7. Aufl., McGraw-Hill, S. 14.95 und 14.96; die Verhinderung eines möglichen Tropfenmitrisses ist auf den Seiten 14.87 bis 14.90 beschrieben. Die Entspannung des präformierten Katalysators kann einstufig oder zweistufig bis zum Erreichen des gewünschten Druckes erfolgen, wobei die Temperatur üblicherweise auf 10 bis 40°C sinkt.

Im Anschluss an die Präformierung gemäß Schritt a) und die Befreiung des Katalysators von überschüssigem Kohlenmonoxid gemäß Schritt b) führt man gemäß Schritt c) des erfindungsgemäßen Verfahrens die asymmetrische Hydrierung des gewählten Substrats in Gegenwart von Wasserstoff mit einem Kohlenmonoxidgehalt von 100 bis 1200 ppm durch.

Die Zugabe von zusätzlichem Kohlenmonoxid zum Reaktionsgemisch der asymmetrische Hydrierung kann auf verschiedene Weise vorgenommen werden: So kann das Kohlenmonoxid beispielsweise dem zur asymmetrischen Hydrierung eingesetzten Wasserstoff beigemischt werden oder auch direkt gasförmig in die Reaktionslösung eindosiert werden. Eine weitere Möglichkeit besteht beispielsweise darin, dem Reaktionsgemisch Verbindungen zuzusetzen, die leicht Kohlenmonoxid freisetzen, wie beispielsweise Formiate oder Oxalyl-Verbindungen.

Der Anteil an Kohlenmonoxid im eingesetzten Wasserstoff beträgt im Rahmen einer bevorzugten Ausführungsform des erfindungsgemäßen Verfahrens etwa 300 bis 1000 ppm, besonders bevorzugt 400 bis 800 ppm.

Die erfindungsgemäße asymmetrische Hydrierung nimmt man vorteilhaft bei einem Druck von etwa 1 bis etwa 300 bar, bevorzugt von etwa 10 bis etwa 100 bar, insbesondere bei etwa 50 bis etwa 100 bar, besonders bevorzugt bei etwa 60 bis etwa 100 bar und einer Temperatur von in der Regel etwa 0°C bis etwa 100°C, bevorzugt etwa 0°C bis etwa 30°C, insbesondere bei etwa 10°C bis etwa 30°C vor.

Die Wahl des zur Durchführung der erfindungsgemäßen asymmetrischen Hydrierung einzusetzenden Lösemittels ist nicht kritisch. Geeignete Lösemittel sind beispielsweise jene zur Durchführung der erfindungsgemäßen Präformierung genannten. Mit besonderem Vorteil führt man die asymmetrische Hydrierung im gleichen Lösemittel durch wie die gegebenenfalls zuvor durchgeführte Präformierung.

Als Reaktionsgefäße zur Durchführung der erfindungsgemäßen asymmetrischen Hydrierung sind prinzipiell all jene geeignet, die Umsetzungen unter den genannten Bedingungen, insbesondere Druck und Temperatur, erlauben und für Hydrierreaktionen geeignet sind, wie beispielsweise Autoklaven, Rohrreaktoren, Blasensäulen, etc.

Führt man die Hydrierung gemäß Schritt c) des erfindungsgemäßen Verfahrens unter Einsatz von hochsiedenden, in der Regel viskosen Lösungsmitteln durch, wie sie beispielsweise vorstehend zum Einsatz im Rahmen der Vorbehandlung des Katalysators gemäß Schritt a) des erfindungsgemäßen Verfahrens beschrieben sind (etwa die genannten Lösungsmittel Octadecanol, Biphenylether, Texanol, Marlotherm^{®}, Oxoöl 9N) oder führt man die Hydrierung ohne zusätzlichen Einsatz von Lösemittel jedoch unter Aufpegelung der als Nebenprodukte in geringem Ausmaß entstehenden Hochsieder (wie beispielsweise Dimere oder Trimere, die durch Reaktionen der Edukte bzw. Produkte und anschließenden Folgereaktionen entstehen) durch, kann es vorteilhaft sein, für guten Gaseintrag und gute Durchmischung von Gasphase und kondensierter Phase zu sorgen. Dies gelingt beispielsweise dadurch, dass man den Hydrierschritt des erfindungsgemäßen Verfahrens in einem Gasumlaufreaktor durchführt. Gasumlaufreaktoren sind dem Fachmann an sich bekannt und beispielsweise in P. Trambouze, J.-P. Euzen, Chemical Reactors, Ed. Technip, 2004, S. 280-283 und P. Zehner, R. Benfer, Chem. Eng. Sci. 1996, 51, 1735-1744 sowie z. B. in der EP 1 140 349 beschrieben.

Bei Einsatz eines wie vorstehend genannten Gasumlaufreaktors hat es sich als besonders vorteilhaft erwiesen, das einzusetzende Gas bzw. Gasgemisch (den Kohlenmonoxid enthaltenden Wasserstoff) parallel zu den in den Reaktor eingetragenen Edukten und/oder dem umlaufenden Reaktionsgemische bzw. dem Katalysator mittels einer einfachen Düse oder einer Zweistoffdüse in den Gasumlaufreaktor einzutragen. Dabei zeichnet sich die Zweistoffdüse dadurch aus, dass in den Reaktor einzubringende Flüssigkeit und Gas durch zwei getrennte ineinander liegenden Röhren unter Druck bis zum Düsenmund gelangen und dort miteinander vereint werden.

Das erfindungsgemäße Verfahren kann mit gutem Erfolg mit und ohne Zugabe von tertiären Aminen durchgeführt werden. Bevorzugt führt man das erfindungsgemäße Verfahren in Abwesenheit, d.h. ohne Zugabe von zusätzlichen tertiären Aminen oder in Anwesenheit nur katalytischer Mengen von zusätzlichen tertiären Aminen durch. Die verwendete Aminmenge kann dabei zwischen 0,5 und 500 mol-Äquivalenten bezogen auf die eingesetzte Menge an Metall, bevorzugt aber 1 bis 100 mol-Äquivalenten bezogen auf die eingesetzte Metallmenge betragen. Die Wahl des tertiären Amins ist nicht kritisch. Neben kurzkettigen Alkylaminen, wie beispielsweise Triethylamin können auch langkettige Alkylamine, wie zum Beispiel Tridodecylamin, verwendet werden. Im Rahmen einer bevorzugten Ausführungsform führt man das erfindungsgemäße Hydrierverfahren in Gegenwart eines tertiären Amins, bevorzugt Tridodecylamin, in einer Menge von etwa 2 bis 30 mol-Äquivalenten, bevorzugt etwa 5 bis 20 mol-Äquivalenten und besonders bevorzugt 5 bis 15 mol-Äquivalenten bezogen auf die Menge an eingesetztem Übergangsmetall ein.

Mit Vorteil bricht man die Reaktion ab, wenn die Zielverbindung in der gewünschten Ausbeute und der gewünschte optischen Aktivität, d.h. mit dem gewünschten Enantiomerenüberschuss (ee) im Reaktionsgemisch vorliegt, wie es der Fachmann durch Routineuntersuchungen beispielsweise mittels chromatographischer Methoden feststellen kann. Üblicherweise ist die Hydrierung nach etwa 1 bis etwa 150 h, oft nach etwa 2 bis etwa 24 h abgeschlossen.

Durch das erfindungsgemäße Verfahren gelingt es, optisch aktive Carbonylverbindungen, insbesondere optisch aktive Aldehyde in hohen Ausbeuten und Enantiomerenüberschüssen bereitzustellen. Üblicherweise erhält man die gewünschten asymmetrisch hydrierten Verbindungen in einem Enantiomerenüberschuss von mindestens 80% ee, oft mit einem Enantiomerenüberschuss von etwa 85 bis etwa 99% ee. Dabei ist zu beachten, dass der maximal erzielbare Enantiomerenüberschuss von der Reinheit des eingesetzten Substrats, insbesondere im Hinblick auf die Isomerenreinheit der zu hydrierenden Doppelbindung, abhängen kann.

Demzufolge eignen sich als Ausgangssubstanzen insbesondere solche, die ein Isomerenverhältnis von mindestens etwa 90:10, bevorzugt mindestens etwa 95:5 bezüglich der E/Z-Doppelbindungsisomere aufweisen.

Das erfindungsgemäße Verfahren zeichnet sich dadurch aus, dass die eingesetzten homogenen Katalysatoren durch das zusätzlich ins Reaktionssystem eingebrachte Kohlenmonoxid stabilisiert werden, wodurch zum einen die Standzeit der Katalysatoren deutlich erhöht wird und zum anderen die Wiederverwendbarkeit der homogenen Katalysatoren ermöglicht wird.

So lässt sich beispielsweise das erhaltene Reaktionsprodukt durch dem Fachmann an sich bekannte Verfahren, wie z.B. durch Destillation, beispielsweise mittels eines Feinfilmverdampfers, Sambays oder dergleichen mehr aus dem Reaktionsgemisch entfernen und der zurückbleibende Katalysator, gegebenenfalls nach abermaliger, wie vorstehend beschriebener Präformierung, im Rahmen weiterer Umsetzungen nutzen.

Das erfindungsgemäße Verfahren wird kontinuierlich betrieben und eignet sich insbesondere für Umsetzungen in technischem Maßstab.
Die erfindungsgemäß durchzuführende Vorbehandlung der Katalysatorvorstufe (Präformierung) gemäß Schritt a) und die eigentliche asymmetrische Hydrierung gemäß Schritt c) werden vorteilhaft in getrennten Reaktionsgefäßen durchgeführt. Bei der Überführung des präformierten Katalysators in den eigentlichen Hydrierrektor, bevorzugt den wie vorstehend beschriebenen Gasumlaufreaktor, kann dann das überschüssige Kohlenmonoxid vom Katalysator, beispielsweise durch Entspannung des zur Präformierung angewendeten Druckes, entfernt werden.

Auch die Hydrierung kann in mehreren, vorzugsweise in zwei oder drei, besonders bevorzugt in zwei, hintereinander geschalteten Hydrierreaktoren erfolgen. Dabei können verschiedenartige oder gleiche Reaktortypen zum Einsatz kommen. In einer bevorzugten Ausführungsform führt man die asymmetrische Hydrierung beispielsweise in einer Kaskade von zwei Gasumlaufreaktoren durch, wobei einer als Hauptreaktor und der zweite als Nachreaktor fungiert. Zur Überführung des Reaktionsgemisches vom Hauptreaktor in den Nachreaktor kann dabei beispielsweise ein gewünschtenfalls so einzustellendes Druckgefälle genutzt werden.

Im Rahmen einer besonders bevorzugten Ausführungsform des erfindungsgemäßen Verfahrens setzt man Neral oder Geranial, bevorzugt Neral, das bis zu etwa 5 mol-%, bevorzugt bis zu etwa 2 mol-% des jeweiligen Doppelbindungsisomeren enthält, zu optisch aktivem Citronellal um. Zur Bildung des Katalysators setzt man bevorzugt eine im Reaktionsgemisch lösliche Verbindung des Rhodium, insbesondere Rh₂(OAc)₄, [Rh(cod)Cl]₂, Rh(CO)₂acac, [Rh(cod)OH]₂, [Rh(cod)OMe]₂, Rh₄(CO)₁₂ oder Rh₆(CO)₁₆ und als chiralen Liganden (R,R)-Chiraphos bzw. (S,S)-Chiraphos ((2R, 3R)-(+)-2,3-bis(Diphenylphosphino)butan bzw. (2S, 3S)-(-)-2,3-bis(Diphenylphosphino)butan) im molaren Verhältnis von etwa 1:1 bis etwa 1:4 bezogen auf Rhodium ein. In einer insbesondere bevorzugten Ausführungsform des erfindungsgemäßen Verfahren setzt man Neral, das bis zu etwa 5 mol-%, bevorzugt bis zu etwa 2 mol-% Geranial enthält in Gegenwart von Rh(OAc)₃, [Rh(cod)Cl]₂, Rh(CO)₂acac, [Rh(cod)OH]₂, [Rh(cod)OMe]₂, Rh₄(CO)₁₂ oder Rh₆(CO)₁₆ und (R,R)-Chiraphos zu D-Citronellal um.

Im Rahmen der bevorzugten Ausführungsform des erfindungsgemäßen Verfahren verzichtet man auf den Zusatz von Lösemitteln und führt die genannten Umsetzungen im umzusetzenden Substrat bzw. dem Produkt und gegebenenfalls in hochsiedenden Nebenprodukten als Lösemedium durch. Erfindungsgemäß ist die kontinuierliche Reaktionsführung unter Wiederverwendung bzw. Rückführung des erfindungsgemäß stabilisierten homogenen Katalysators.

Ein weiterer Aspekt der vorliegenden Erfindung betrifft ein Verfahren zur Herstellung von optisch aktivem Menthol unter Einsatz von nach dem erfindungsgemäßen Verfahren hergestelltem optisch aktivem Citronellal. Die Herstellung von optisch aktivem Menthol ausgehend von optisch aktivem Citronellal ist bekannt. Ein Schlüsselschritt ist hierbei die Cyclisierung von optisch aktivem Citronellal zu optisch aktivem Isopulegol, wie beispielsweise in der EP-A 1 225 163 beschrieben.

Das erfindungsgemäß hergestellte optisch aktive Citronellal lässt sich, wie nachstehend schematisch für die Herstellung von L-Menthol der Formel (XIII) dargestellt, in Gegenwart einer geeigneten Säure, insbesondere einer Lewis-Säure zu L-Isopulegol der Formel (XII) cyclisieren und anschließend nach dem Fachmann bekannten Verfahren, beispielsweise durch katalytische Hydrierung wie beispielsweise in der J. Am. Chem. Soc. 1984, 106, 5208-5217 oder Synthesis 1991, 665-680. beschrieben, zum L-Menthol hydrieren.

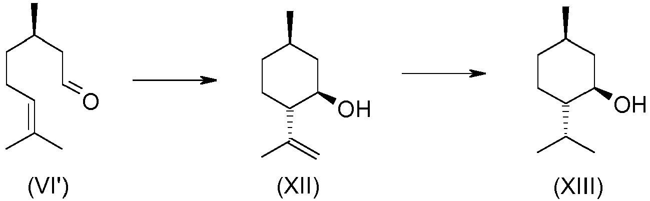

Ein weiterer Aspekt der vorliegenden Erfindung betrifft demgemäss ein Verfahren zur Herstellung von optisch aktivem Menthol, umfassend die Schritte
i) Herstellung von optisch aktivem Citronellal gemäß dem vorstehend beschriebenen erfindungsgemäßen Verfahren ,
ii) Cyclisierung des so hergestellten optisch aktiven Citronellals zu optisch aktivem Isopulegol in Gegenwart einer Lewis-Säure und
iii) Hydrierung des so hergestellten optisch aktiven Isopulegols zu optisch aktivem Menthol.

Im Rahmen einer diesbezüglich bevorzugten Ausführungsform stellt man gemäß Schritt i) R-Citronellal durch erfindungsgemäße Hydrierung von Geranial oder Neral, bevorzugt Neral her, cyclisiert das so erhaltene R-Citronellal gemäß Schritt ii) zu L-Isopulegol und hydriert gemäß Schritt iii) das so erhaltene L-Isopulegol zu L-Menthol.

Die folgenden Beispiele dienen der Verdeutlichung der Erfindung, ohne sie in irgend einer Weise zu beschränken:

### Beispiel 1:

Kontinuierlich betriebene asymmetrische Hydrierung von cis-Citral mit gezielter Verringerung des CO-Gehaltes nach der Präformierung

In einer kontinuierlich betriebenen Anlage wurde eine Lösung von insgesamt 10,4 g Rh(CO)₂acac und insgesamt 34,5 g (R,R)-Chiraphos in Toluol (660 ml) eingefüllt. Die Gasmischung wurde im Präformierungsreaktor bei einem Druck von 80 bar und einer Temperatur von 60°C auf ein Verhältnis von Wasserstoff zu Kohlenmonoxid (H₂:CO) von 1:1 eingestellt. Der Austrag des Präformierungsreaktors wurde in einen Hochdruckabscheider auf Normaldruck entspannt und anschließend auf 80 bar in den Hydrierreaktor komprimiert. In den Hydrierreaktor wurden bei 20°C 158 Nl/h H₂ (mit 400 ppm CO) eingeleitet, so dass sich im Abgas des Hydrierreaktors ein CO-Wert von 550 ppm einstellte.

Bei einem Zulauf von 100 g/h cis-Citral (Neral) mit einem Gehalt von 98 Gew.-% wurde eine produkthaltige Fraktion im Vakuum kontinuierlich so abdestilliert, dass der Anlageninhalt nahezu konstant blieb. Es wurde mit diesen Einstellungen innerhalb von 8 Tagen 110,9 mol (17,1 kg) D-Citronellal isoliert. Die Ausbeute an D-Citronellal betrug bezogen auf das eingesetzte cis-Citral 93%.

### Vergleichsbeispiel:

Kontinuierlich betriebene asymmetrische Hydrierung von cis-Citral bei verminderter CO-Konzentration bei der Präformierung und ohne Verringerung des CO-Gehaltes nach der Präformierung

In einer kontinuierlich betriebenen Anlage wurde eine Lösung von insgesamt 10,4 g Rh(CO)₂acac und insgesamt 34,5 g (R, R)-Chiraphos in Toluol (660 ml) eingefüllt. Die Gasmischung im Präformierungsreaktor wurde bei einem Druck von 105 bar und einer Temperatur von 60°C auf ein Verhältnis von 90:10 H₂:CO (35.1 Nl/h) eingestellt. Der Austrag des Präformierungsreaktors wurde ohne vorige Druckentspannung direkt auf 80 bar in den Hydrierreaktor entspannt. In den Hydrierreaktor wurden bei 20°C 150 Nl/h H₂ eingeleitet, so dass sich im Abgas des Hydrierreaktors ein CO-Wert von etwa 800 ppm einstellte.

Bei einem Zulauf von 77 g/h cis-Citral (Neral) mit einem Gehalt von 98 Gew.-% wurde eine produkthaltige Fraktion im Vakuum kontinuierlich so abdestilliert, dass der Anlageninhalt nahezu konstant blieb. Es wurden mit diesen Einstellungen innerhalb von 8 Tagen 82,1 mol D-Citronellal isoliert.
Die Ausbeute an D-Citronellal betrug bezogen auf das eingesetzte cis-Citral lediglich 86%.

### Beispiel 2:

Kontinuierlich betriebene asymmetrische Hydrierung von cis-Citral mit erhöhtem CO-Gehalt im Hydrierreaktor

In einer kontinuierlich betriebenen Anlage wurde eine Lösung von insgesamt 10,4 g Rh(CO)₂acac und insgesamt 25,8 g (R,R)-Chiraphos in Toluol (etwa 600 ml) eingefüllt.

Das Verhältnis von Wasserstoff zu Kohlenmonoxid (H₂:CO) wurde im Präformierungsreaktor bei einem Druck von 80 bar und einer Temperatur von 60°C auf 1:1 eingestellt. Der Austrag des Präformierungsreaktors wurde in einen Hochdruckabscheider auf Normaldruck entspannt und anschließend auf 80 bar in den Hydrierreaktor verdichtet. In den Hydrierreaktoren wurden bei 20°C 60 NI/h H₂ (mit 245 ppm CO) eingeleitet, so dass sich im Abgas des Hydrierreaktors ein CO-Wert von etwa 600 ppm einstellte.

Bei einem Zulauf von 100 g/h cis-Citral (Neral) mit einem Gehalt von 98 Gew.-% wurde eine produkthaltige Fraktion im Vakuum kontinuierlich so abdestilliert, dass der Anlageninhalt nahezu konstant blieb. Es wurde mit diesen Einstellungen innerhalb von 5 Tagen 2,88 mol (443,5 g) D-Citronellal isoliert. Die Ausbeute an D-Citronellal betrug bezogen auf das eingesetzte cis-Citral 92%.

### Beispiel 3:

Unter denselben Grundeinstellungen wurde der Hydrierreaktor mit 60,3 NI/h H₂ mit 265 ppm CO beschickt, wobei sich im Abgas des Hydrierkatalysators ein CO-Wert von etwa 1300 ppm einstellte. Auf diese Weise wurden innerhalb von 5 Tagen 2,87 mol (441,9 g) D-Citronellal isoliert. Die Ausbeute an D-Citronellal betrug bezogen auf das eingesetzte cis-Citral 90%.

## Patentansprüche

1. Kontinuierliches Verfahren zur Herstellung optisch aktiver Carbonylverbindungen der Formel (I) 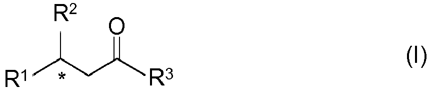 wobei die Reste
R¹, R² jeweils für einen unverzweigten, verzweigten oder cyclischen Alkylrest mit 1 bis 25 Kohlenstoffatomen, der gegebenenfalls eine oder mehrere ethylenische Doppelbindungen und/oder einen oder mehrere gleiche oder verschiedene Substituenten ausgewählt aus der Gruppe der Substituenten OR⁴, NR⁵R⁶, Halogen, C₆-C₁₀-Aryl und C₃-C₉-Hetaryl tragen kann und der gemeinsam mit R³ einen 5 bis 25-gliedrigen Ring bilden kann, steht, mit der Maßgabe, dass R¹ und R² verschieden sind
R³ für Wasserstoff oder einen unverzweigten, verzweigten oder cyclischen Alkylrest mit 1 bis 25 Kohlenstoffatomen, der gegebenenfalls eine oder mehrere ethylenische Doppelbindungen und/oder eine oder mehrere gleiche oder verschiedene Substituenten ausgewählt aus der Gruppe der Substituenten OR⁴, NR⁵R⁶, Halogen, C₆-C₁₀-Aryl und C₃-C₉-Hetaryl tragen kann, oder für OR⁷ oder NR⁸R⁹ steht
wobei
R⁴, R⁵, R⁶ jeweils unabhängig voneinander Wasserstoff, C₁-C₆-Alkyl, C₆-C₁₀-Aryl, C₇-C₁₂-Aralkyl oder C₇-C₁₂-Alkylaryl bedeuten und
R⁵ und R⁶ gemeinsam auch eine Alkylenkette mit 2 bis 5 Kohlenstoffatomen, die durch N oder O unterbrochen sein kann, bedeuten können und
R⁷ für einen unverzweigten, verzweigten oder cyclischen Alkylrest mit 1 bis 25 Kohlenstoffatomen, der gegebenenfalls eine oder mehrere ethylenische Doppelbindungen und eine oder mehrere gleiche oder verschiedene Substituenten ausgewählt aus der Gruppe der Substituenten OR⁴, NR⁵R⁶, Halogen, C₆-C₁₀-Aryl und C₃-C₉-Hetaryl tragen kann, steht und gemeinsam mit R¹ oder R² einen 5 bis 25-gliedrigen Ring bilden kann und
R⁸ für einen unverzweigten, verzweigten oder cyclischen Alkylrest mit 1 bis 25 Kohlenstoffatomen, der gegebenenfalls eine oder mehrere ethylenische Doppelbindungen und eine oder mehrere gleiche oder verschiedene Substituenten ausgewählt aus der Gruppe der Substituenten OR⁴, NR⁵R⁶, Halogen, C₆-C₁₀-Aryl und C₃-C₉-Hetaryl tragen kann, stehen und gemeinsam mit R¹, R² oder R⁹ einen 5 bis 25-gliedrigen Ring bilden kann und
R⁹ für Wasserstoff, C₁-C₆-Alkyl, C₆-C₁₀-Aryl, C₇-C₁₂-Aralkyl oder C₇-C₁₂-Alkylaryl steht oder zusammen mit R⁸ einen 5 bis 25-gliedrigen Ring bilden kann und
* ein asymmetrisches Kohlenstoffatom bezeichnet,
durch asymmetrische Hydrierung a,β-ungesättigter Aldehyde oder Ketone der Formel (II) 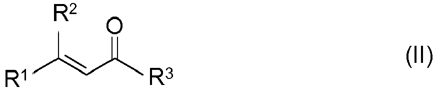 wobei die Reste R¹ bis R³ die oben angegebene Bedeutung besitzen,
in Gegenwart von im Reaktionsgemisch löslichen, optisch aktiven Übergangsmetall-Katalysatoren, die mindestens einen Kohlenmonoxid-Liganden aufweisen, wobei man zur Herstellung des jeweils einzusetzenden optisch aktiven, mindestens einen Kohlenmonoxid-Liganden aufweisenden Katalysators eine Katalysator-Vorstufe mit einem Kohlenmonoxid und Wasserstoff enthaltenden Gasgemisch vorbehandelt und die asymmetrische Hydrierung in Gegenwart von dem Reaktionsgemisch zusätzlich zugeführtem Kohlenmonoxid durchführt, indem man gemäß der aufeinanderfolgenden Schritte
a) die Vorbehandlung der Katalysator-Vorstufe mit einem Gasgemisch umfassend 30 bis 70 Vol.-% Kohlenmonoxid, 30 bis 70 Vol-% Wasserstoff und 0 bis 5 Vol.-% weiterer Gase, wobei sich die genannten Volumenanteile zu 100 Vol.-% ergänzen, bei einem Druck von 50 bis 90 bar durchführt,
b) von dem so erhaltenen Katalysator vor Einsatz in der asymmetrischen Hydrierung überschüssiges Kohlenmonoxid abtrennt, indem man den gemäß Schritt a) vorbehandelten Katalysator auf einen Druck im Bereich von 1 bis 3 bar entspannt und
c) die asymmetrische Hydrierung in Gegenwart von Wasserstoff mit einem Kohlenmonoxidgehalt von 100 bis 1200 ppm durchführt,
und das erhaltene Reaktionsprodukt vom Reaktionsgemisch entfernt wird und der zurückbleibende Katalysator nach der Vorbehandlung gemäß Schritt a) zu weiteren Umsetzungen genutzt wird.

2. Verfahren nach Anspruch 1 zur Herstellung optisch aktiver Aldehyde der Formel (III) 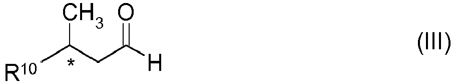 wobei
R¹⁰ für einen unverzweigten oder verzweigten Alkylrest mit 2 bis 25 Kohlenstoffatomen, der gegebenenfalls 1 bis 5 ethylenische Doppelbindungen aufweisen kann, steht und
* ein asymmetrisches Kohlenstoffatom bezeichnet,
durch asymmetrische Hydrierung a,β-ungesättigter Aldehyde der Formel (IV) oder (V) 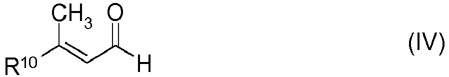 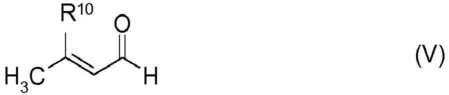 wobei der Rest R¹⁰ die oben angegebene Bedeutung besitzt.

3. Verfahren nach Anspruch 2 zur Herstellung von optisch aktivem Citronellal der Formel (VI) 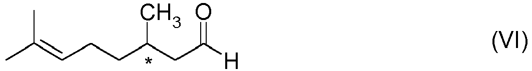 durch asymmetrische Hydrierung von Neral der Formel (VII) oder Geranial der Formel (VIII)

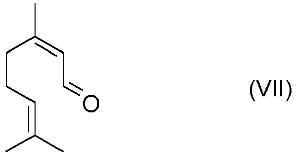

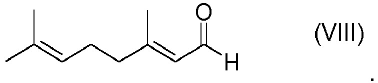

4. Verfahren nach Anspruch 3 zur Herstellung von D-Citronellal durch asymmetrische Hydrierung von Neral.

5. Verfahren nach einem der Ansprüche 1 bis 4, **dadurch gekennzeichnet, dass** man eine Übergangsmetall-Katalysator-Vorstufe einsetzt, die erhältlich ist durch Reaktion mindestens einer im Reaktionsgemisch löslichen Übergangsmetall-Verbindung mit einem optisch aktiven Liganden, der mindestens ein Phosphor- und/oder Arsenatom aufweist.

6. Verfahren nach Anspruch 5, **dadurch gekennzeichnet, dass** man als optisch aktiven Liganden eine Verbindung der allgemeinen Formeln (IX), (X) oder (XI) 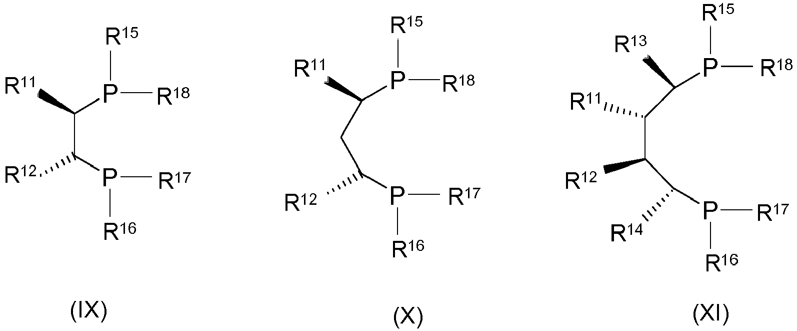 in denen
R¹¹, R¹²: jeweils unabhängig voneinander für einen unverzweigten, verzweigten oder cyclischen Alkylrest mit 1 bis 20 Kohlenstoffatomen, der gegebenenfalls eine oder mehrere, in der Regel 1 bis etwa 4, ethylenische Doppelbindungen und/oder einen oder mehrere, in der Regel 1 bis etwa 4, gleiche oder verschiedene Substituenten ausgewählt aus der Gruppe der Substituenten OR¹⁹, NR²⁰R²¹, Halogen, C₆-C₁₀-Aryl und C₃-C₉-Hetaryl tragen kann und R¹¹ und R¹² gemeinsam einen 4 bis 20-gliedrigen Ring bilden können, der eines oder mehrere, in der Regel 1 oder 2 O-Atome beinhalten kann, stehen und
R¹³, R¹⁴: jeweils unabhängig voneinander Wasserstoff oder geradkettiges oder verzweigtes C₁- bis C₄-Alkyl bedeuten und
R¹⁵, R¹⁶, R¹⁷ R¹⁸: jeweils für C₆- bis C₁₀-Aryl, das gegebenenfalls einen oder mehrere, in der Regel 1 bis 8, bevorzugt 1 bis 4 Substituenten ausgewählt aus der Gruppe der Substituenten C₁- bis C₄-Alkyl, C₆- bis C₁₀-Aryl, C₁- bis C₄-Alkoxy und Amino tragen kann, stehen und
R¹⁹, R²⁰, R²¹: jeweils unabhängig voneinander Wasserstoff, C₁-C₄-Alkyl, C₆-C₁₀-Aryl, C₇-C₁₂-Aralkyl oder C₇-C₁₂-Alkylaryl bedeuten, wobei
R²⁰, R²¹: gemeinsam auch eine Alkylenkette mit 2 bis 5 Kohlenstoffatomen, die durch N oder O unterbrochen sein kann, bedeuten können
einsetzt.

7. Verfahren nach Anspruch 5 oder 6, **dadurch gekennzeichnet, dass** die Übergangsmetallverbindung eine Verbindung eines Metalls der VIII. Nebengruppe des Periodensystems der Elemente ist.

8. Verfahren nach einem der Ansprüche 5 bis 7, **dadurch gekennzeichnet, dass** man eine Verbindung der Metalle Rhodium oder Iridium einsetzt.

9. Verfahren nach einem der Ansprüche 5 bis 8, **dadurch gekennzeichnet, dass** die Übergangsmetallverbindung eine Rhodium-Verbindung ist.

10. Verfahren nach einem der Ansprüche 5 bis 9, **dadurch gekennzeichnet, dass** man einen optisch aktiven Liganden der Formel (IX) einsetzt, wobei den Resten R¹¹, R¹² und R¹⁵ bis R¹⁸ die vorstehend genannten Bedeutungen zukommen.

11. Verfahren nach einem der Ansprüche 5 bis 10, dass man als optisch aktiven Liganden den Liganden der Formel (1) (S,S-Chiraphos) oder ent-(1) (R,R-Chiraphos) 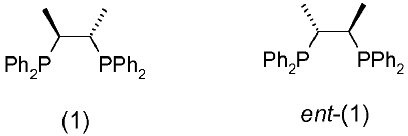 einsetzt.

12. Verfahren nach einem der Ansprüche 1 bis 11, **dadurch gekennzeichnet, dass** man die asymmetrische Hydrierung bei einem Druck von 10 bis 100 bar durchführt.

13. Verfahren nach einem der Ansprüche 1 bis 12, **dadurch gekennzeichnet, dass** man die Vorbehandlung der Katalysator-Vorstufe gemäß Schritt a) mit einem Gasgemisch umfassend 40 bis 60 Vol.-% Kohlenmonoxid, 60 bis 40 Vol-% Wasserstoff und 0 bis 5 Vol.-% weiterer Gase, wobei sich die genannten Volumenanteile zu 100 Vol.-% ergänzen, durchführt.

14. Verfahren nach einem der Ansprüche 1 bis 13, **dadurch gekennzeichnet, dass** man die asymmetrische Hydrierung gemäß Schritt c) in Gegenwart von Wasserstoff mit einem Kohlenmonoxidgehalt von 400 bis 800 ppm durchführt.

15. Verfahren nach einem der Ansprüche 1 bis 14, **dadurch gekennzeichnet, dass** man die asymmetrische Hydrierung gemäß Schritt c) in einem Gasumlaufreaktor durchführt.

16. Verfahren nach Anspruch 15, **dadurch gekennzeichnet, dass** man die umzusetzenden α,β-ungesättigten Aldehyde oder Ketone und den Wasserstoff mittels einer Zweistoffdüse in den Gasumlaufreaktor einträgt.

17. Verfahren zur Herstellung von optisch aktivem Menthol umfassend die Schritte
i) Herstellung von optisch aktivem Citronellal nach einem der Ansprüche 1 bis 16,
ii) Cyclisierung des so hergestellten optisch aktiven Citronellals zu optisch aktivem Isopulegol in Gegenwart einer Lewis-Säure und
iii) Hydrierung des so hergestellten optisch aktiven Isopulegols zu optisch aktivem Menthol.

## Claims

1. A continuous process for preparing optically active carbonyl compounds of the formula (I) 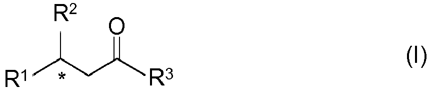 where
the R¹, R² radicals are each an unbranched, branched or cyclic alkyl radical which has from 1 to 25 carbon atoms and may optionally bear one or more ethylenic double bonds and/or one or more identical or different substituents selected from the group of the substituents OR⁴, NR⁵R⁶, halogen, C₆-C₁₀-aryl and C₃-C₉-hetaryl, and which, together with R³, may form a 5- to 25-membered ring, with the proviso that R¹ and R² are different,
the R³ radical is hydrogen or an unbranched, branched or cyclic alkyl radical which has from 1 to 25 carbon atoms and may optionally bear one or more ethylenic double bonds and/or one or more identical or different substituents selected from the group of the substituents OR⁴, NR⁵R⁶, halogen, C₆-C₁₀-aryl and C₃-C₉-hetaryl, or OR⁷ or NR⁸R⁹,
where
R⁴, R⁵, R⁶ are each independently hydrogen, C₁-C₆-alkyl, C₆-C₁₀-aryl, C₇-C₁₂-aralkyl or C₇-C₁₂-alkylaryl and
R⁵ and R⁶ together may also be an alkylene chain which has from 2 to 5 carbon atoms and may be interrupted by N or 0 and
R⁷ is an unbranched, branched or cyclic alkyl radical which has from 1 to 25 carbon atoms and may optionally bear one or more ethylenic double bonds and one or more identical or different substituents selected from the group of the substituents OR⁴, NR⁵R⁶, halogen, C₆-C₁₀-aryl and C₃-C₉-hetaryl, and, together with R¹ or R², may form a 5- to 25-membered ring and
R⁸ is an unbranched, branched or cyclic alkyl radical which has from 1 to 25 carbon atoms and may optionally bear one or more ethylenic double bonds and one or more identical or different substituents selected from the group of the substituents OR⁴, NR⁵R⁶, halogen, C₆-C₁₀-aryl and C₃-C₉-hetaryl, and, together with R¹, R² or R⁹, may form a 5- to 25-membered ring and
R⁹ is hydrogen, C₁-C₆-alkyl, C₆-C₁₀-aryl, C₇-C₁₂-aralkyl or C₇-C₁₂-alkylaryl, or, together with R⁸, may form a 5- to 25-membered ring and
* designates an asymmetric carbon atom,
by asymmetrically hydrogenating α,β-unsaturated aldehydes or ketones of the formula (II) 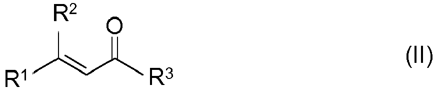 where the R¹ to R³ radicals are each as defined above,
in the presence of optically active transition metal catalysts which are soluble in the reaction mixture and have at least one carbon monoxide ligand, the optically active catalyst which has at least one carbon monoxide ligand and is to be used in each case being prepared by pretreating a catalyst precursor with a gas mixture comprising carbon monoxide and hydrogen and the asymmetric hydrogenation being performed in the presence of carbon monoxide supplied additionally to the reaction mixture, which comprises the successive steps of
a) performing the pretreatment of the catalyst precursor with a gas mixture comprising from 30 to 70% by volume of carbon monoxide, from 30 to 70% by volume of hydrogen and from 0 to 5% by volume of further gases, the proportions by volume mentioned adding up to 100% by volume, at a pressure of from 50 to 90 bar,
b) removing excess carbon monoxide from the catalyst thus obtained before use in the asymmetric hydrogenation by decompressing the catalyst pretreated in step a) to a pressure in the range of from 1 to 3 bar and
c) performing the asymmetric hydrogenation in the presence of hydrogen with a carbon monoxide content of from 100 to 1200 ppm,
and removing the reaction product obtained from the reaction mixture and utilizing the remaining catalyst for further reactions after the pretreatment according to step a).

2. The process according to claim 1 for preparing optically active aldehydes of the formula (III) 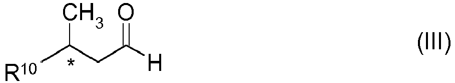 where
R¹⁰ is an unbranched or branched alkyl radical which has from 2 to 25 carbon atoms and may optionally have from 1 to 5 ethylenic double bonds and
* designates an asymmetric carbon atom, by asymmetrically hydrogenating α,β-unsaturated aldehydes of the formula (IV) or (V) 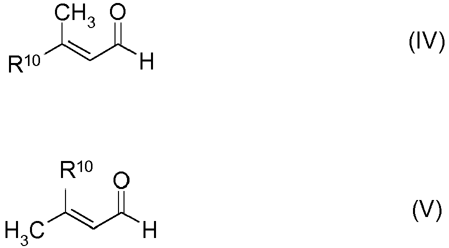
where the R¹⁰ radical is as defined above.

3. The process according to claim 2 for preparing optically active citronellal of the formula (VI) 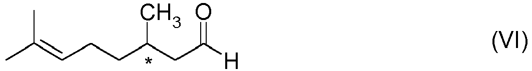 by asymmetrically hydrogenating neral of the formula (VII) or geranial of the formula (VIII)

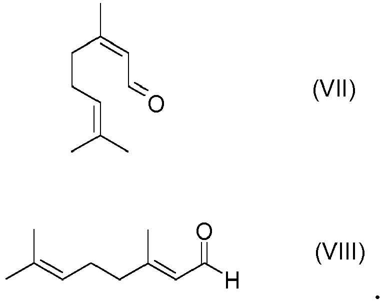

4. The process according to claim 3 for preparing D-citronellal by asymmetrically hydrogenating neral.

5. The process according to any of claims 1 to 4, wherein a transition metal catalyst precursor which is obtainable by reacting at least one transition metal compound which is soluble in the reaction mixture and has an optically active ligand which has at least one phosphorus and/or arsenic atom is used.

6. The process according to claim 5, wherein the optically active ligand used is a compound of the general formula (IX), (X) or (XI) 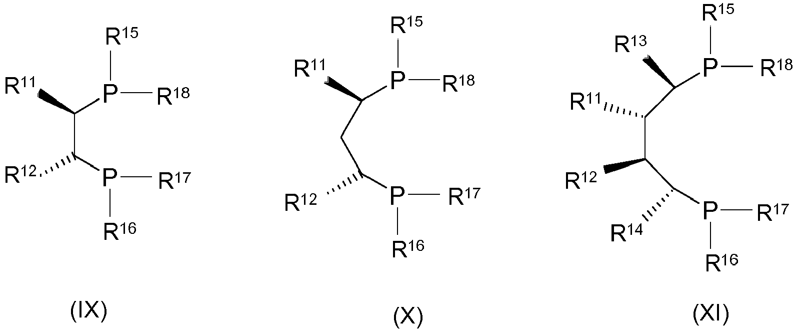 in which
R¹¹, R¹²: are each independently an unbranched, branched or cyclic alkyl radical which has from 1 to 20 carbon atoms and may optionally bear one or more, generally from 1 to about 4, ethylenic double bonds and/or one or more, generally from 1 to about 4, identical or different substituents selected from the group of the substituents OR¹⁹, NR²⁰R²¹, halogen, C₆-C₁₀-aryl and C₃-C₉-hetaryl, and R¹¹ and R¹² together may form a 4- to 20-membered ring which may include one or more, generally 1 or 2, oxygen atoms, and
R¹³, R¹⁴: are each independently hydrogen or straight-chain or branched C₁- to C₄-alkyl and
R¹⁵, R¹⁶, R¹⁷, R¹⁸: are each C₆- to C₁₀-aryl which may optionally bear one or more, generally from 1 to 8, preferably from 1 to 4, substituents selected from the group of the substituents C₁- to C₄-alkyl, C₆- to C₁₀-aryl, C₁-to C₄-alkoxy and amino, and
R¹⁹, R²⁰, R²¹: are each independently hydrogen, C₁-C₄-alkyl, C₆-C₁₀-aryl, C₇-C₁₂-aralkyl or C₇-C₁₂-alkylaryl, where
R²⁰, R²¹: together may also be an alkylene chain which has from 2 to 5 carbon atoms and may be interrupted by N or O.

7. The process according to claim 5 or 6, wherein the transition metal compound is a compound of a metal of transition group VIII of the Periodic Table of the Elements.

8. The process according to any of claims 5 to 7, wherein a compound of the metals rhodium or iridium is used.

9. The process according to any of claims 5 to 8, wherein the transition metal compound is a rhodium compound.

10. The process according to any of claims 5 to 9, wherein an optically active ligand of the formula (IX) where the R¹¹, R¹² and R¹⁵ to R¹⁸ radicals are each as defined above is used.

11. The process according to any of claims 5 to 10, that the optically active ligand used is the ligand of the formula (1) (S,S-chiraphos) or ent-(1) (R,R-chiraphos)

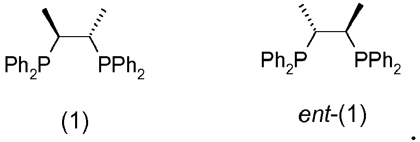

12. The process according to any of claims 1 to 11, wherein the asymmetric hydrogenation is performed at a pressure of from 10 to 100 bar.

13. The process according to any of claims 1 to 12, wherein the pretreatment of the catalyst precursor in step a) is performed with a gas mixture comprising from 40 to 60% by volume of carbon monoxide, from 60 to 40% by volume of hydrogen and from 0 to 5% by volume of further gases, where the proportions by volume mentioned add up to 100% by volume.

14. The process according to any of claims 1 to 13, wherein the asymmetric hydrogenation in step c) is performed in the presence of hydrogen having a carbon monoxide content of from 400 to 800 ppm.

15. The process according to any of claims 1 to 14, wherein the asymmetric hydrogenation in step c) is performed in a gas circulation reactor.

16. The process according to claim 15, wherein the α,β-unsaturated aldehydes or ketones to be converted and the hydrogen are introduced into the gas circulation reactor by means of a two-substance nozzle.

17. A process for preparing optically active menthol, comprising the steps of
i) preparing optically active citronellal according to any of claims 1 to 16,
ii) cyclizing the optically active citronellal thus prepared to optically active isopulegol in the presence of a Lewis acid and
iii) hydrogenating the optically active isopulegol thus prepared to optically active menthol.

## Revendications

1. Procédé continu de fabrication de composés de carbonyle optiquement actifs de formule (I) 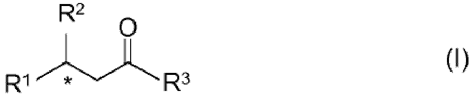 dans laquelle les radicaux
R¹, R² représentent chacun un radical alkyle non ramifié, ramifié ou cyclique de 1 à 25 atomes de carbone, qui peut éventuellement porter une ou plusieurs doubles liaisons éthyléniques et/ou un ou plusieurs substituants identiques ou différents choisis dans le groupe constitué par les substituants OR⁴, NR⁵R⁶, halogène, aryle en C₆-C₁₀ et hétaryle en C₃-C₉, et qui peut former conjointement avec R³ un cycle de 5 à 25 éléments, à condition que R¹ et R² soient différents,
R³ représente l'hydrogène ou un radical alkyle non ramifié, ramifié ou cyclique de 1 à 25 atomes de carbone, qui peut éventuellement porter une ou plusieurs doubles liaisons éthyléniques et/ou un ou plusieurs substituants identiques ou différents choisis dans le groupe constitué par les substituants OR⁴, NR⁵R⁶, halogène, aryle en C₆-C₁₀ et hétaryle en C₃-C₉, ou représente OR⁷ ou NR⁸R⁹,
R⁴, R⁵, R⁶ signifiant chacun indépendamment les uns des autres hydrogène, alkyle en C₁-C₆, aryle en C₆-C₁₀, aralkyle en C₇-C₁₂ ou alkylaryle en C₇-C₁₂, et
R⁵ et R⁶ pouvant également signifier ensemble une chaîne alkylène de 2 à 5 atomes de carbone, qui peut être interrompue par N ou 0, et
R⁷ représentant un radical alkyle non ramifié, ramifié ou cyclique de 1 à 25 atomes de carbone, qui peut éventuellement porter une ou plusieurs doubles liaisons éthyléniques et un ou plusieurs substituants identiques ou différents choisis dans le groupe constitué par les substituants OR⁴, NR⁵R⁶, halogène, aryle en C₆-C₁₀ et hétaryle en C₃-C₉, et pouvant former conjointement avec R¹ ou R² un cycle de 5 à 25 éléments,
et
R⁸ représentant un radical alkyle non ramifié, ramifié ou cyclique de 1 à 25 atomes de carbone, qui peut éventuellement porter une ou plusieurs doubles liaisons éthyléniques et un ou plusieurs substituants identiques ou différents choisis dans le groupe constitué par les substituants OR⁴, NR⁵R⁶, halogène, aryle en C₆-C₁₀ et hétaryle en C₃-C₉, et pouvant former conjointement avec R¹, R² ou R⁹ un cycle de 5 à 25 éléments, et
R⁹ représentant hydrogène, alkyle en C₁-C₆, aryle en C₆-C₁₀, aralkyle en C₇-C₁₂ ou alkylaryle en C₇-C₁₂, ou pouvant former conjointement avec R⁸ un cycle de 5 à 25 éléments, et
* désigne un atome de carbone asymétrique,
par hydrogénation asymétrique d'aldéhydes ou de cétones α,β-insaturés de formule (II) 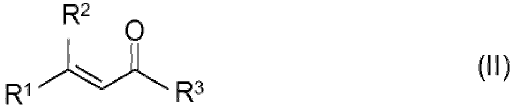 dans laquelle les radicaux R¹ à R³ ont la signification indiquée précédemment,
en présence de catalyseurs de métaux de transition optiquement actifs, solubles dans le mélange réactionnel, qui comprennent au moins un ligand monoxyde de carbone, un précurseur de catalyseur étant prétraité avec un mélange gazeux contenant du monoxyde de carbone et de l'hydrogène pour la fabrication du catalyseur optiquement actif comprenant au moins un ligand monoxyde de carbone à utiliser à chaque fois, et l'hydrogénation asymétrique étant réalisée en présence de monoxyde de carbone en outre introduit dans le mélange réactionnel, selon les étapes successives suivantes :
a) le prétraitement du précurseur de catalyseur avec un mélange gazeux comprenant 30 à 70 % en volume de monoxyde de carbone, 30 à 70 % en volume d'hydrogène et 0 à 5 % en volume d'autres gaz, la somme des proportions volumiques indiquées étant de 100 % en volume, est réalisé à une pression de 50 à 90 bar,
b) le monoxyde de carbone en excès est séparé du catalyseur ainsi obtenu avant l'utilisation dans l'hydrogénation asymétrique, par détente du catalyseur prétraité selon l'étape a) à une pression dans la plage allant de 1 à 3 bar, et
c) l'hydrogénation asymétrique est réalisée en présence d'hydrogène à une teneur en monoxyde de carbone de 100 à 1 200 ppm,
et le produit de réaction obtenu est séparé du mélange réactionnel et le catalyseur restant est utilisé après le prétraitement selon l'étape a) pour des réactions ultérieures.

2. Procédé selon la revendication 1 pour la fabrication d'aldéhydes optiquement actifs de formule (III) 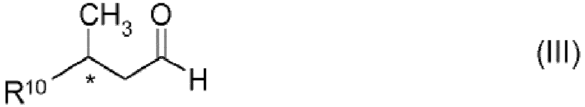 dans laquelle
R¹⁰ représente un radical alkyle non ramifié ou ramifié de 2 à 25 atomes de carbone, qui peut éventuellement comprendre 1 à 5 doubles liaisons éthyléniques, et
* désigne un atome de carbone asymétrique,
par hydrogénation asymétrique d'aldéhydes α,β-insaturés de formule (IV) ou (V) 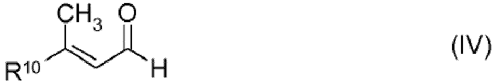 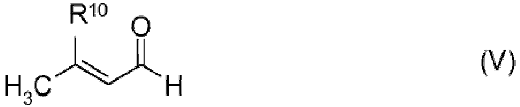 dans lesquelles le radical R¹⁰ a la signification indiquée précédemment.

3. Procédé selon la revendication 2, pour la fabrication de citronellal optiquement actif de formule (VI) 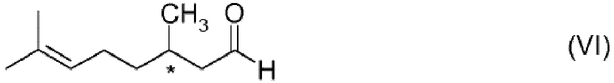 par hydrogénation asymétrique de néral de formule (VII) ou de géranial de formule (VIII)

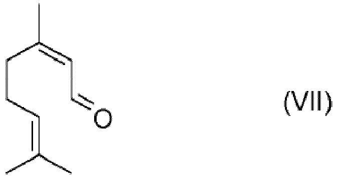

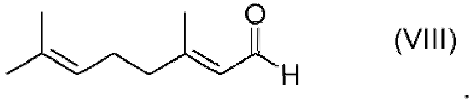

4. Procédé selon la revendication 3 pour la fabrication de D-citronellal par hydrogénation asymétrique de néral.

5. Procédé selon l'une quelconque des revendications 1 à 4, **caractérisé en ce qu'**un précurseur de catalyseur de métal de transition est utilisé, qui peut être obtenu par réaction d'au moins un composé de métal de transition soluble dans le mélange réactionnel avec un ligand optiquement actif, qui comprend au moins un atome de phosphore et/ou d'arsenic.

6. Procédé selon la revendication 5, **caractérisé en ce qu'**en tant que ligand optiquement actif, un composé de formule générale (IX), (X) ou (XI) 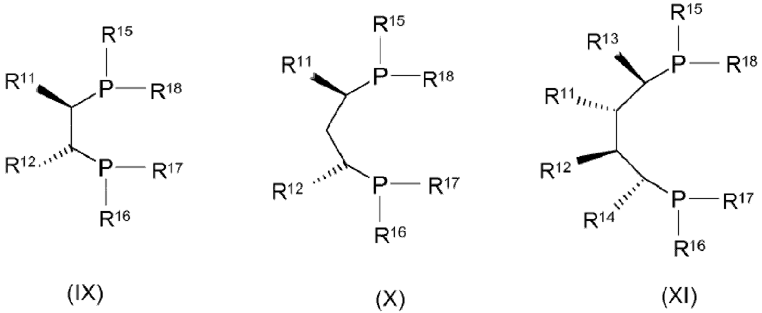 dans lesquelles
R¹¹, R¹² représentent chacun indépendamment l'un de l'autre un radical alkyle non ramifié, ramifié ou cyclique de 1 à 20 atomes de carbone, qui peut éventuellement porter une ou plusieurs, généralement 1 à environ 4, doubles liaisons éthyléniques, et/ou un ou plusieurs, généralement 1 à environ 4, substituants identiques ou différents choisis dans le groupe constitué par les substituants OR¹⁹, NR²⁰R²¹, halogène, aryle en C₆-C₁₀ et hétaryle en C₃-C₉, et R¹¹ et R¹² peuvent former ensemble un cycle de 4 à 20 éléments, qui peut contenir un ou plusieurs, généralement 1 ou 2, atomes 0, et
R¹³, R¹⁴ signifient chacun indépendamment l'un de l'autre hydrogène ou alkyle en C₁ à C₄ linéaire ou ramifié, et
R¹⁵, R¹⁶, R¹⁷, R¹⁸ représentent chacun aryle en C₆ à C₁₀, qui peut éventuellement porter un ou plusieurs, généralement 1 à 8, de préférence 1 à 4, substituants choisis dans le groupe constitué par les substituants alkyle en C₁ à C₄, aryle en C₆ à C₁₀, alcoxy en C₁ à C₄ et amino, et
R¹⁹, R²⁰, R²¹ signifient chacun indépendamment les uns des autres hydrogène, alkyle en C₁-C₄, aryle en C₆-C₁₀, aralkyle en C₇-C₁₂ ou alkylaryle en C₇-C₁₂, R²⁰, R²¹ pouvant également signifier ensemble une chaîne alkylène de 2 à 5 atomes de carbone, qui peut être interrompue par N ou 0,
est utilisé.

7. Procédé selon la revendication 5 ou 6, **caractérisé en ce que** le composé de métal de transition est un composé d'un métal du groupe de transition VIII du tableau périodique des éléments.

8. Procédé selon l'une quelconque des revendications 5 à 7, **caractérisé en ce qu'**un composé du métal rhodium ou iridium est utilisé.

9. Procédé selon l'une quelconque des revendications 5 à 8, **caractérisé en ce que** le composé de métal de transition est un composé de rhodium.

10. Procédé selon l'une quelconque des revendications 5 à 9, **caractérisé en ce qu'**un ligand optiquement actif de formule (IX) est utilisé, les radicaux R¹¹, R¹² et R¹⁵ à R¹⁸ ayant les significations indiquées précédemment.

11. Procédé selon l'une quelconque des revendications 5 à 10, qu'en tant que ligand optiquement actif, le ligand de formule (1) (S,S-chiraphos) ou ent-(1) (R,R-chiraphos) 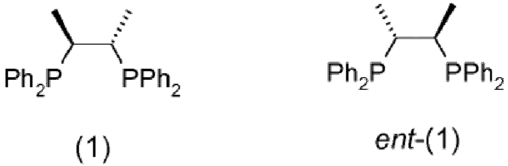 est utilisé.

12. Procédé selon l'une quelconque des revendications 1 à 11, **caractérisé en ce que** l'hydrogénation asymétrique est réalisée à une pression de 10 à 100 bar.

13. Procédé selon l'une quelconque des revendications 1 à 12, **caractérisé en ce que** le prétraitement du précurseur de catalyseur selon l'étape a) est réalisé avec un mélange gazeux comprenant 40 à 60 % en volume de monoxyde de carbone, 60 à 40 % en volume d'hydrogène et 0 à 5 % en volume d'autres gaz, la somme des proportions volumiques étant de 100 % en volume.

14. Procédé selon l'une quelconque des revendications 1 à 13, **caractérisé en ce que** l'hydrogénation asymétrique selon l'étape c) est réalisée en présence d'hydrogène ayant une teneur en monoxyde de carbone de 400 à 800 ppm.

15. Procédé selon l'une quelconque des revendications 1 à 14, **caractérisé en ce que** l'hydrogénation asymétrique selon l'étape c) est réalisée dans un réacteur à circulation de gaz.

16. Procédé selon la revendication 15, **caractérisé en ce que** les aldéhydes ou cétones α,β-insaturés et l'hydrogène à mettre en réaction sont introduits dans le réacteur à circulation de gaz par une buse à deux composants.

17. Procédé de fabrication de menthol optiquement actif, comprenant les étapes suivantes :
i) la fabrication de citronellal optiquement actif selon l'une quelconque des revendications 1 à 16,
ii) la cyclisation du citronellal optiquement actif ainsi fabriqué en isopulégol optiquement actif en présence d'un acide de Lewis, et
iii) l'hydrogénation de l'isopulégol optiquement actif ainsi fabriqué en menthol optiquement actif.
